# EUROPÄISCHE PATENTSCHRIFT

(11) **EP 1 294 913 B1**
(45) Veröffentlichungstag und Bekanntmachung des Hinweises auf die Patenterteilung: **12.03.2008**
(21) Anmeldenummer: 01943549.4
(22) Anmeldetag: 28.06.2001
(51) Int. Cl.: C12N 15/82, C12N 15/62, C12N 9/02, C12N 9/90, A01H 5/00

(54) **VERÄNDERUNG DES GEHALTS AN FEINCHEMIKALIEN IN ORGANISMEN DURCH GENETISCHE VERÄNDERUNG DES SHIKIMATWEGES**
CHANGING THE FINE CHEMICAL CONTENT IN ORGANISMS BY GENETICALLY MODIFYING THE SHIKIMATE PATHWAY
MODIFICATION DE LA TENEUR EN PRODUITS CHIMIQUES FINS DANS LES ORGANISMES PAR MODIFICATION GENETIQUE DU CHEMIN DU SHIKIMATE

(30) Priorität: 29.06.2000 DE 10030647; 21.12.2000 DE 10064454
(43) Veröffentlichungstag der Anmeldung: 26.03.2003
(73) Patentinhaber: Sungene GmbH & Co. KGaA, 06466 Gatersleben (DE)
(72) Erfinder: BADUR, Ralf, 67117 Limburgerhof (DE); GEIGER, Michael, 06484 Quedlinburg (DE); KUNZE, Irene, 06466 Gatersleben (DE); SOMMER, Susanne, 65719 Hofheim (DE)
(74) Vertreter: Meyer, Udo
(86) Internationale Anmeldenummer: PCT/EP2001/007391
(87) Internationale Veröffentlichungsnummer: WO 2002/000901

(56) Entgegenhaltungen:
- EP-A- 0 264 914
- WO-A-00/08169
- WO-A-91/04263
- EBERHARD J ET AL: "Cytosolic and plastidic chorismate mutase isozymes from Arabidopsis thaliana: Molecular characterization and enzymatic properties." PLANT JOURNAL, Bd. 10, Nr. 5, 1996, Seiten 815-821, XP002180796 ISSN: 0960-7412 in der Anmeldung erwähnt
- HUDSON G S ET AL: "NUCLEOTIDE SEQUENCE AND TRANSCRIPTION OF THE PHENYLALANINE AND TYROSINE OPERONS OF ESCHERICHIA-COLI K-12" JOURNAL OF MOLECULAR BIOLOGY, Bd. 180, Nr. 4, 1984, Seiten 1023-1052, XP001030690 ISSN: 0022-2836

### SEQUENZPROTOKOLL

<110> SunGene GmbH & Co. KGaA
<120> Veränderung des Gehalts an Feinchemikalien in Organismen durch genetische Veränderung des Shikimatweges
<130> 0817/780/2000
<140>
   <141>
<160> 14
<170> PatentIn Vers. 2.0
<210> 1
   <211> 1238
   <212> DNA
   <213> Escherichia coli
<220>
   <221> CDS
   <222> (25)..(1143)
<400> 1
   cccgggtggc ttaagaggtt tatt atg gtt gct gaa ttg acc gca tta cgc     51
                             Met Val Ala Glu Leu Thr Ala Leu Arg
                               1               5
   gat caa att gat gaa gtc gat aaa gcg ctg ctg aat tta tta gcg aag    99
   Asp Gln Ile Asp Glu Val Asp Lys Ala Leu Leu Asn Leu Leu Ala Lys
    10                  15                  20                  25
   cgt ctg gaa ctg gtt gct gaa gtg ggc gag gtg aaa agc cgc ttt gga    147
   Arg Leu Glu Leu Val Ala Glu Val Gly Glu Val Lys Ser Arg Phe Gly
                    30                  35                  40
   ctg cct att tat gtt ccg gag cgc gag gca tct atg ttg gcc tcg cgt    195
   Leu Pro Ile Tyr Val Pro Glu Arg Glu Ala Ser Met Leu Ala Ser Arg
                45                  50                  55
   cgt gca gag gcg gaa gct ctg ggt gta ccg cca gat ctg att gag gat    243
   Arg Ala Glu Ala Glu Ala Leu Gly Val Pro Pro Asp Leu Ile Glu Asp
            60                  65                  70
   gtt ttg cgt cgg gtg atg cgt gaa tct tac tcc agt gaa aac gac aaa    291
   Val Leu Arg Arg Val Met Arg Glu Ser Tyr Ser Ser Glu Asn Asp Lys
        75                  80                  85
   gga ttt aaa aca ctt tgt ccg tca ctg cgt ccg gtg gtt atc gtc ggc     339
   Gly Phe Lys Thr Leu Cys Pro Ser Leu Arg Pro Val Val Ile Val Gly
    90                 95                 100                 105
   ggt ggc ggt cag atg gga cgc ctg ttc gag aag atg ctg acc ctc tcg     387
   Gly Gly Gly Gln Met Gly Arg Leu Phe Glu Lys Met Leu Thr Leu Ser
                   110                 115                 120
   ggt tat cag gtg cgg att ctg gag caa cat gac tgg gat cga gcg gct     435
   Gly Tyr Gln Val Arg Ile Leu Glu Gln His Asp Trp Asp Arg Ala Ala
               125                 130                 135
   gat att gtt gcc gat gcc gga atg gtg att gtt agt gtg cca atc cac     483
   Asp Ile Val Ala Asp Ala Gly Met Val Ile Val Ser Val Pro Ile His
           140                 145                 150
   gtt act gag caa gtt att ggc aaa tta ccg cct tta ccg aaa gat tgt     531
   Val Thr Glu Gln Val Ile Gly Lys Leu Pro Pro Leu Pro Lys Asp Cys
       155                 160                 165
   att ctg gtc gat ctg gca tca gtg aaa aat ggg cca tta cag gcc atg     579
   Ile Leu Val Asp Leu Ala Ser Val Lys Asn Gly Pro Leu Gln Ala Met
   170                 175                 180                 185
   ctg gtg gcg cat gat ggt ccg gtg ctg ggg cta cac ccg atg ttc ggt     627
   Leu Val Ala His Asp Gly Pro Val Leu Gly Leu His Pro Met Phe Gly
                   190                 195                 200
   ccg gac agc ggt agc ctg gca aag caa gtt gtg gtc tgg tgt gat gga     675
   Pro Asp Ser Gly Ser Leu Ala Lys Gln Val Val Val Trp Cys Asp Gly
               205                 210                 215
   cgt aaa ccg gaa gca tac caa tgg ttt ctg gag caa att cag gtc tgg     723
   Arg Lys Pro Glu Ala Tyr Gln Trp Phe Leu Glu Gln Ile Gln Val Trp
           220                 225                 230
   ggc gct cgg ctg cat cgt att agc gcc gtc gag cac gat cag aat atg     771
   Gly Ala Arg Leu His Arg Ile Ser Ala Val Glu His Asp Gln Asn Met
       235                 240                 245
   gcg ttt att cag gca ctg cgc cac ttt gct act ttt gct tac ggg ctg     819
   Ala Phe Ile Gln Ala Leu Arg His Phe Ala Thr Phe Ala Tyr Gly Leu
   250                 255                 260                 265
   cac ctg gca gaa gaa aat gtt cag ctt gag caa ctt ctg gcg ctc tct     867
   His Leu Ala Glu Glu Asn Val Gln Leu Glu Gln Leu Leu Ala Leu Ser
                   270                 275                 280
   tcg ccg att tac cgc ctt gag ctg gcg atg gtc ggg cga ctg ttt gct     915
   Ser Pro Ile Tyr Arg Leu Glu Leu Ala Met Val Gly Arg Leu Phe Ala
               285                 290                 295
   cag gat ccg cag ctt tat gcc gac atc att atg tcg tca gag cgt aat     963
   Gln Asp Pro Gln Leu Tyr Ala Asp Ile Ile Met Ser Ser Glu Arg Asn
           300                 305                 310
   ctg gcg tta atc aaa cgt tac tat aag cgt ttc ggc gag gcg att gag    1011
   Leu Ala Leu Ile Lys Arg Tyr Tyr Lys Arg Phe Gly Glu Ala Ile Glu
       315                 320                 325
   ttg ctg gag cag ggc gat aag cag gcg ttt att gac agt ttc cgc aag    1059
   Leu Leu Glu Gln Gly Asp Lys Gln Ala Phe Ile Asp Ser Phe Arg Lys
   330                 335                 340                 345
   gtg gag cac tgg ttc ggc gat tac gca cag cgt ttt cag agt gaa agc    1107
   Val Glu His Trp Phe Gly Asp Tyr Ala Gln Arg Phe Gln Ser Glu Ser
                   350                 355                 360
   cgc gtg tta ttg cgt cag gcg aat gac aat cgc cag taataatcca         1153
   Arg Val Leu Leu Arg Gln Ala Asn Asp Asn Arg Gln
               365                 370
   gtgccggatg attcacatca tccggcacct tttcatcagg ttggatcaac aggcactacg 1213
   ttctcacttg ggtaacagcg tcgac                                        1238
<210> 2
   <211> 373
   <212> PRT
   <213> Escherichia coli
<400> 2
   Met Val Ala Glu Leu Thr Ala Leu Arg Asp Gln Ile Asp Glu Val Asp
   1               5                   10                  15
   Lys Ala Leu Leu Asn Leu Leu Ala Lys Arg Leu Glu Leu Val Ala Glu
               20                  25                  30
   Val Gly Glu Val Lys Ser Arg Phe Gly Leu Pro Ile Tyr Val Pro Glu
           35                  40                  45
   Arg Glu Ala Ser Met Leu Ala Ser Arg Arg Ala Glu Ala Glu Ala Leu
       50                  55                  60
   Gly Val Pro Pro Asp Leu Ile Glu Asp Val Leu Arg Arg Val Met Arg
   65                  70                  75                  80
   Glu Ser Tyr Ser Ser Glu Asn Asp Lys Gly Phe Lys Thr Leu Cys Pro
                   85                  90                  95
   Ser Leu Arg Pro Val Val Ile Val Gly Gly Gly Gly Gln Met Gly Arg
               100                 105                 110
   Leu Phe Glu Lys Met Leu Thr Leu Ser Gly Tyr Gln Val Arg Ile Leu
           115                 120                 125
   Glu Gln His Asp Trp Asp Arg Ala Ala Asp Ile Val Ala Asp Ala Gly
       130                 135                 140
   Met Val Ile Val Ser Val Pro Ile His Val Thr Glu Gln Val Ile Gly
   145                 150                 155                 160
   Lys Leu Pro Pro Leu Pro Lys Asp Cys Ile Leu Val Asp Leu Ala Ser
                   165                 170                 175
   Val Lys Asn Gly Pro Leu Gln Ala Met Leu Val Ala His Asp Gly Pro
               180                 185                 190
   Val Leu Gly Leu His Pro Met Phe Gly Pro Asp Ser Gly Ser Leu Ala
           195                 200                 205
   Lys Gln Val Val Val Trp Cys Asp Gly Arg Lys Pro Glu Ala Tyr Gln
       210                 215                 220
   Trp Phe Leu Glu Gln Ile Gln Val Trp Gly Ala Arg Leu His Arg Ile
   225                 230                 235                 240
   Ser Ala Val Glu His Asp Gln Asn Met Ala Phe Ile Gln Ala Leu Arg
                   245                 250                 255
   His Phe Ala Thr Phe Ala Tyr Gly Leu His Leu Ala Glu Glu Asn Val
               260                 265                 270
   Gln Leu Glu Gln Leu Leu Ala Leu Ser Ser Pro Ile Tyr Arg Leu Glu
           275                 280                 285
   Leu Ala Met Val Gly Arg Leu Phe Ala Gln Asp Pro Gln Leu Tyr Ala
       290                 295                 300
   Asp Ile Ile Met Ser Ser Glu Arg Asn Leu Ala Leu Ile Lys Arg Tyr
   305                 310                 315                 320
   Tyr Lys Arg Phe Gly Glu Ala Ile Glu Leu Leu Glu Gln Gly Asp Lys
                   325                 330                 335
   Gln Ala Phe Ile Asp Ser Phe Arg Lys Val Glu His Trp Phe Gly Asp
               340                 345                 350
   Tyr Ala Gln Arg Phe Gln Ser Glu Ser Arg Val Leu Leu Arg Gln Ala
           355                 360                 365
   Asn Asp Asn Arg Gln
   370
<210> 3
   <211> 1006
   <212> DNA
   <213> Arabidopsis thaliana
<220>
   <221> CDS
   <222> (64)..(858)
<400> 3
   ctttagcatt gaggaagaag aagaagaaag cttcattttt ccaggggata cagttgaagc 60
   ggc atg gca aga gtc ttc gaa tcg gat tcg ggt tct ggt tgt tcc aat    108
       Met Ala Arg Val Phe Glu Ser Asp Ser Gly Ser Gly Cys Ser Asn
       1               5                   10                  15
   gta ctg agt ctt gac tta atc aga gaa tcg ttg att agg caa gaa gac    156
   Val Leu Ser Leu Asp Leu Ile Arg Glu Ser Leu Ile Arg Gln Glu Asp
                   20                  25                  30
   acc atc gtc ttc agc ttg atc gag aga gct aag ttt cca ctc aat tct    204
   Thr Ile Val Phe Ser Leu Ile Glu Arg Ala Lys Phe Pro Leu Asn Ser
               35                  40                  45
   cct gct ttc gag gaa tct cgt tgt cta gat tct gga agt ttc tct tct    252
   Pro Ala Phe Glu Glu Ser Arg Cys Leu Asp Ser Gly Ser Phe Ser Ser
           50                  55                  60
   ctc act gag ttt ttc gtc aga gag aca gaa atc atc caa gct aag gta    300
   Leu Thr Glu Phe Phe Val Arg Glu Thr Glu Ile Ile Gln Ala Lys Val
       65                  70                  75
   gga aga tat gaa tac ccg gaa gag aat cct ttc ttc ctt gag aac att    348
   Gly Arg Tyr Glu Tyr Pro Glu Glu Asn Pro Phe Phe Leu Glu Asn Ile
   80                  85                  90                  95
   cct cac tcg gtt ttt cct acg cac aaa tat cca tcg gct ttg cac cct    396
   Pro His Ser Val Phe Pro Thr His Lys Tyr Pro Ser Ala Leu His Pro
                   100                 105                 110
   aag gct cta tct gtt aac att aac aaa caa atc tgg gat att tac ttt    444
   Lys Ala Leu Ser Val Asn Ile Asn Lys Gln Ile Trp Asp Ile Tyr Phe
               115                 120                 125
   aaa gaa ttg ctt cct ttg ttt gtc aaa cct ggc gat gat ggc aac tat    492
   Lys Glu Leu Leu Pro Leu Phe Val Lys Pro Gly Asp Asp Gly Asn Tyr
           130                 135                 140
   cca tca act gct gct agt gat ctc gcc tgt tta caa gct ctt tcg aga   540
   Pro Ser Thr Ala Ala Ser Asp Leu Ala Cys Leu Gln Ala Leu Ser Arg
       145                 150                 155
   agg att cac tac ggt aaa ttt gta gct gag gtc aaa ttc aga gat gct   588
   Arg Ile His Tyr Gly Lys Phe Val Ala Glu Val Lys Phe Arg Asp Ala
   160                 165                 170                 175
   cca caa gat tac gag cct gcg att cgc gct cag gat aga gag gct ttg   636
   Pro Gln Asp Tyr Glu Pro Ala Ile Arg Ala Gln Asp Arg Glu Ala Leu
                   180                 185                 190
   atg aag ctg ttg acg ttt gag aaa gta gaa gaa atg gtt aag aag aga   684
   Met Lys Leu Leu Thr Phe Glu Lys Val Glu Glu Met Val Lys Lys Arg
               195                 200                 205
   gtg cag aag aaa gca gaa acg ttt gga caa gaa gta aaa ttc aac tct   732
   Val Gln Lys Lys Ala Glu Thr Phe Gly Gln Glu Val Lys Phe Asn Ser
           210                 215                 220
   ggc tat ggc gat gag agt aag aag aag tat aaa gtg gat cca ttg ctt   780
   Gly Tyr Gly Asp Glu Ser Lys Lys Lys Tyr Lys Val Asp Pro Leu Leu
       225                 230                 235
   gcc tct cgc atc tac ggg gaa tgg ctt atc cct ctc act aag ctc gtt   828
   Ala Ser Arg Ile Tyr Gly Glu Trp Leu Ile Pro Leu Thr Lys Leu Val
   240                 245                 250                 255
   gag gtt gag tat ctt cta cgt cgt ctc gat tgaatattat ttgtatccaa     878
   Glu Val Glu Tyr Leu Leu Arg Arg Leu Asp
                   260                 265
   atctggccct gttaaagtgg gccttaagtt tttaagtggg cctgttgata tttgtcagga 938
   tatgatagaa taattgaatg aagcaacaca gtcatcacta ttttaaattt tgtaagatat 998
   tttaagga                                                          1006
<210> 4
   <211> 265
   <212> PRT
   <213> Arabidopsis thaliana
<400> 4
   Met Ala Arg Val Phe Glu Ser Asp Ser Gly Ser Gly Cys Ser Asn Val
   1               5                   10                  15
   Leu Ser Leu Asp Leu Ile Arg Glu Ser Leu Ile Arg Gln Glu Asp Thr
               20                  25                  30
   Ile Val Phe Ser Leu Ile Glu Arg Ala Lys Phe Pro Leu Asn Ser Pro
           35                  40                  45
   Ala Phe Glu Glu Ser Arg Cys Leu Asp Ser Gly Ser Phe Ser Ser Leu
       50                  55                  60
   Thr Glu Phe Phe Val Arg Glu Thr Glu Ile Ile Gln Ala Lys Val Gly
   65                  70                  75                  80
   Arg Tyr Glu Tyr Pro Glu Glu Asn Pro Phe Phe Leu Glu Asn Ile Pro
                   85                  90                  95
   His Ser Val Phe Pro Thr His Lys Tyr Pro Ser Ala Leu His Pro Lys
               100                 105                 110
   Ala Leu Ser Val Asn Ile Asn Lys Gln Ile Trp Asp Ile Tyr Phe Lys
           115                 120                 125
   Glu Leu Leu Pro Leu Phe Val Lys Pro Gly Asp Asp Gly Asn Tyr Pro
       130                 135                 140
   Ser Thr Ala Ala Ser Asp Leu Ala Cys Leu Gln Ala Leu Ser Arg Arg
   145                 150                 155                 160
   Ile His Tyr Gly Lys Phe Val Ala Glu Val Lys Phe Arg Asp Ala Pro
                   165                 170                 175
   Gln Asp Tyr Glu Pro Ala Ile Arg Ala Gln Asp Arg Glu Ala Leu Met
               180                 185                 190
   Lys Leu Leu Thr Phe Glu Lys Val Glu Glu Met Val Lys Lys Arg Val
           195                 200                 205
   Gln Lys Lys Ala Glu Thr Phe Gly Gln Glu Val Lys Phe Asn Ser Gly
       210                 215                 220
   Tyr Gly Asp Glu Ser Lys Lys Lys Tyr Lys Val Asp Pro Leu Leu Ala
   225                 230                 235                 240
   Ser Arg Ile Tyr Gly Glu Trp Leu Ile Pro Leu Thr Lys Leu Val Glu
                   245                 250                 255
   Val Glu Tyr Leu Leu Arg Arg Leu Asp
               260                 265
<210> 5
   <211> 993
   <212> DNA
   <213> Artificial Sequence
<220>
   <223> Die Beschreibung von Knstliche Sequenz: Chimäre
   Nukleins.: Transitpept. der plastid.
   Chorismatm.+kod. Sequenz der cytosolischen
   Chorismatmutase
<220>
   <221> CDS
   <222> (1)..(993)
<400> 5
   atg aga tcg tct tgt tgc tcc tct gcg att ggt ggg ttc ttc gac cat    48
   Met Arg Ser Ser Cys Cys Ser Ser Ala Ile Gly Gly Phe Phe Asp His
     1               5                  10                  15
   cga cgt gaa tta tca acc tca aca ccc att tcc act ctt ctt cct ctt    96
   Arg Arg Glu Leu Ser Thr Ser Thr Pro Ile Ser Thr Leu Leu Pro Leu
                20                  25                  30
   cca tca acc aaa tct tct ttc tct gtt cgt tgt tct ctt cct cag cca   144
   Pro Ser Thr Lys Ser Ser Phe Ser Val Arg Cys Ser Leu Pro Gln Pro
            35                  40                  45
   tca aag cca cgc tct gga acc agc tct gtt cac gcc gtt atg aca ctc   192
   Ser Lys Pro Arg Ser Gly Thr Ser Ser Val His Ala Val Met Thr Leu
        50                  55                  60
   gcc atg gca aga gtc ttc gaa tcg gat tcg ggt tct ggt tgt tcc aat   240
   Ala Met Ala Arg Val Phe Glu Ser Asp Ser Gly Ser Gly Cys Ser Asn
    65                  70                  75                  80
   gta ctg agt ctt gac tta atc aga gaa tcg ttg att agg caa gaa gac   288
   Val Leu Ser Leu Asp Leu Ile Arg Glu Ser Leu Ile Arg Gln Glu Asp
                    85                  90                  95
   acc atc gtc ttc agc ttg atc gag aga gct aag ttt cca ctc aat tct   336
   Thr Ile Val Phe Ser Leu Ile Glu Arg Ala Lys Phe Pro Leu Asn Ser
               100                 105                 110
   cct gct ttc gag gaa tct cgt tgt cta gat tct gga agt ttc tct tct   384
   Pro Ala Phe Glu Glu Ser Arg Cys Leu Asp Ser Gly Ser Phe Ser Ser
           115                 120                 125
   ctc act gag ttt ttc gtc aga gag aca gaa atc atc caa gct aag gta   432
   Leu Thr Glu Phe Phe Val Arg Glu Thr Glu Ile Ile Gln Ala Lys Val
       130                 135                 140
   gga aga tat gaa tac ccg gaa gag aat cct ttc ttc ctt gag aac att     480
   Gly Arg Tyr Glu Tyr Pro Glu Glu Asn Pro Phe Phe Leu Glu Asn Ile
   145                 150                 155                 160
   cct cac tcg gtt ttt cct acg cac aaa tat cca tcg gct ttg cac cct     528
   Pro His Ser Val Phe Pro Thr His Lys Tyr Pro Ser Ala Leu His Pro
                   165                 170                 175
   aag gct cta tct gtt aac att aac aaa caa atc tgg gat att tac ttt     576
   Lys Ala Leu Ser Val Asn Ile Asn Lys Gln Ile Trp Asp Ile Tyr Phe
               180                 185                 190
   aaa gaa ttg ctt cct ttg ttt gtc aaa cct ggc gat gat ggc aac tat     624
   Lys Glu Leu Leu Pro Leu Phe Val Lys Pro Gly Asp Asp Gly Asn Tyr
           195                 200                 205
   cca tca act gct gct agt gat ctc gcc tgt tta caa gct ctt tcg aga     672
   Pro Ser Thr Ala Ala Ser Asp Leu Ala Cys Leu Gln Ala Leu Ser Arg
       210                 215                 220
   agg att cac tac ggt aaa ttt gta gct gag gtc aaa ttc aga gat gct     720
   Arg Ile His Tyr Gly Lys Phe Val Ala Glu Val Lys Phe Arg Asp Ala
   225                 230                 235                 240
   cca caa gat tac gag cct gcg att cgc gct cag gat aga gag gct ttg     768
   Pro Gln Asp Tyr Glu Pro Ala Ile Arg Ala Gln Asp Arg Glu Ala Leu
                   245                 250                 255
   atg aag ctg ttg acg ttt gag aaa gta gaa gaa atg gtt aag aag aga     816
   Met Lys Leu Leu Thr Phe Glu Lys Val Glu Glu Met Val Lys Lys Arg
               260                 265                 270
   gtg cag aag aaa gca gaa acg ttt gga caa gaa gta aaa ttc aac tct     864
   Val Gln Lys Lys Ala Glu Thr Phe Gly Gln Glu Val Lys Phe Asn Ser
           275                 280                 285
   ggc tat ggc gat gag agt aag aag aag tat aaa gtg gat cca ttg ctt     912
   Gly Tyr Gly Asp Glu Ser Lys Lys Lys Tyr Lys Val Asp Pro Leu Leu
       290                 295                 300
   gcc tct cgc atc tac ggg gaa tgg ctt atc cct ctc act aag ctc gtt     960
   Ala Ser Arg Ile Tyr Gly Glu Trp Leu Ile Pro Leu Thr Lys Leu Val
   305                 310                 315                 320
   gag gtt gag tat ctt cta cgt cgt ctc gat tga                         993
   Glu Val Glu Tyr Leu Leu Arg Arg Leu Asp
                   325                 330
<210> 6
   <211> 330
   <212> PRT
   <213> Artificial Sequence
<400> 6
   Met Arg Ser Ser Cys Cys Ser Ser Ala Ile Gly Gly Phe Phe Asp His
   1               5                   10                  15
   Arg Arg Glu Leu Ser Thr Ser Thr Pro Ile Ser Thr Leu Leu Pro Leu
               20                  25                  30
   Pro Ser Thr Lys Ser Ser Phe Ser Val Arg Cys Ser Leu Pro Gln Pro
           35                  40                  45
   Ser Lys Pro Arg Ser Gly Thr Ser Ser Val His Ala Val Met Thr Leu
       50                  55                  60
   Ala Met Ala Arg Val Phe Glu Ser Asp Ser Gly Ser Gly Cys Ser Asn
   65                  70                  75                  80
   Val Leu Ser Leu Asp Leu Ile Arg Glu Ser Leu Ile Arg Gln Glu Asp
                   85                  90                  95
   Thr Ile Val Phe Ser Leu Ile Glu Arg Ala Lys Phe Pro Leu Asn Ser
               100                 105                 110
   Pro Ala Phe Glu Glu Ser Arg Cys Leu Asp Ser Gly Ser Phe Ser Ser
           115                 120                 125
   Leu Thr Glu Phe Phe Val Arg Glu Thr Glu Ile Ile Gln Ala Lys Val
       130                 135                 140
   Gly Arg Tyr Glu Tyr Pro Glu Glu Asn Pro Phe Phe Leu Glu Asn Ile
   145                 150                 155                 160
   Pro His Ser Val Phe Pro Thr His Lys Tyr Pro Ser Ala Leu His Pro
                   165                 170                 175
   Lys Ala Leu Ser Val Asn Ile Asn Lys Gln Ile Trp Asp Ile Tyr Phe
               180                 185                 190
   Lys Glu Leu Leu Pro Leu Phe Val Lys Pro Gly Asp Asp Gly Asn Tyr
           195                 200                 205
   Pro Ser Thr Ala Ala Ser Asp Leu Ala Cys Leu Gln Ala Leu Ser Arg
       210                 215                 220
   Arg Ile His Tyr Gly Lys Phe Val Ala Glu Val Lys Phe Arg Asp Ala
   225                 230                 235                 240
   Pro Gln Asp Tyr Glu Pro Ala Ile Arg Ala Gln Asp Arg Glu Ala Leu
                   245                 250                 255
   Met Lys Leu Leu Thr Phe Glu Lys Val Glu Glu Met Val Lys Lys Arg
               260                 265                 270
   Val Gln Lys Lys Ala Glu Thr Phe Gly Gln Glu Val Lys Phe Asn Ser
           275                 280                 285
   Gly Tyr Gly Asp Glu Ser Lys Lys Lys Tyr Lys Val Asp Pro Leu Leu
       290                 295                 300
   Ala Ser Arg Ile Tyr Gly Glu Trp Leu Ile Pro Leu Thr Lys Leu Val
   305                 310                 315                 320
   Glu Val Glu Tyr Leu Leu Arg Arg Leu Asp
                   325                 330
<210> 7
   <211> 218
   <212> DNA
   <213> Arabidopsis thaliana
<220>
   <221> CDS
   <222> (20)..(217)
<400> 7
   ggtaccggcg tcattgttg atg aga tcg tct tgt tgc tcc tct gcg att ggt   52
                        Met Arg Ser Ser Cys Cys Ser Ser Ala Ile Gly
                         1               5                  10
   ggg ttc ttc gac cat cga cgt gaa tta tca acc tca aca ccc att tcc    100
   Gly Phe Phe Asp His Arg Arg Glu Leu Ser Thr Ser Thr Pro Ile Ser
               15                  20                  25
   act ctt ctt cct ctt cca tca acc aaa tct tct ttc tct gtt cgt tgt    148
   Thr Leu Leu Pro Leu Pro Ser Thr Lys Ser Ser Phe Ser Val Arg Cys
           30                  35                  40
   tct ctt cct cag cca tca aag cca cgc tct gga acc agc tct gtt cac    196
   Ser Leu Pro Gln Pro Ser Lys Pro Arg Ser Gly Thr Ser Ser Val His
       45                  50                  55
   gcc gtt atg aca ctc gcc atg g                                      218
   Ala Val Met Thr Leu Ala Met
    60                  65
<210> 8
   <211> 66
   <212> PRT
   <213> Arabidopsis thaliana
<400> 8
   Met Arg Ser Ser Cys Cys Ser Ser Ala Ile Gly Gly Phe Phe Asp His
   1 5 10 15
   Arg Arg Glu Leu Ser Thr Ser Thr Pro Ile Ser Thr Leu Leu Pro Leu
   20 25 30
   Pro Ser Thr Lys Ser Ser Phe Ser Val Arg Cys Ser Leu Pro Gln Pro
   35 40 45
   Ser Lys Pro Arg Ser Gly Thr Ser Ser Val His Ala Val Met Thr Leu
   50 55 60
   Ala Met
   65
<210> 9
   <211> 29
   <212> DNA
   <213> Artificial Sequence
<220>
   <223> Die Beschreibung von Knstliche Sequenz:Primer
<220>
   <221> primer_bind
   <222> (1)..(29)
<400> 9
   aagtcgacgc tgttacccaa gtgagaacg 29
<210> 10
   <211> 30
   <212> DNA
   <213> Artificial Sequence
<220>
   <223> Die Beschreibung von Knstliche Sequenz:Primer
<220>
   <221> primer_bind
   <222> (1)..(30)
<400> 10
   aacccgggtg gcttaagagg tttattatgg 30
<210> 11
   <211> 28
   <212> DNA
   <213> Artificial Sequence
<220>
   <223> Die Beschreibung von Knstliche Sequenz:Primer
<220>
   <221> primer_bind
   <222> (1)..(28)
<400> 11
   ggtaccggcg tcattgttga tgagatcg 28
<210> 12
   <211> 24
   <212> DNA
   <213> Artificial Sequence
<220>
   <223> Die Beschreibung von Knstliche Sequenz: Primer
<220>
   <221> primer_bind
   <222> (1)..(24)
<400> 12
   ccatggtggc gagtgtcata acgg 24
<210> 13
   <211> 27
   <212> DNA
   <213> Artificial Sequence
<220>
   <221> primer_bind
   <222> (1)..(27)
<220>
   <223> Die Beschreibung von Knstliche Sequenz:Primer
<400> 13
   gtcgactcaa tcgagacgac gtagaag 27 <210> 14
   <211> 25
   <212> DNA
   <213> Artificial Sequence
<220>
   <223> Die Beschreibung von Knstliche Sequenz:Primer
<220>
   <221> primer_bind
   <222> (1)..(25)
<400> 14
   ccatgggcaa gagtcttcga atcgg 25

## Beschreibung

Die vorliegende Erfindung betrifft ein Verfahren zur Herstellung von Feinchemikalien, insbesondere Vitamin E, durch Kultivierung von Organismen, insbesondere Pflanzen, die gegenüber dem Wildtyp einen genetisch veränderten Shikimatweg aufweisen.

Organismen, insbesondere Pflanzen, weisen eine Reihe von Stoffwechselprodukten auf, die als Feinchemikalien einen hohen wirtschaftlichen Wert haben. Als Feinchemikalien sind beispielsweise aromatische Aminosäuren, Salicylsäurederivate, Phenylpropanoide, Flavonoide, Stilbene, Xanthone und Chinone, insbesondere die gemischten Prenyllipid-Verbindungen mit Vitamin E oder Vitamin K Aktivität zu nennen.

In biotechnologischen Verfahren zur Herstellung von Feinchemikalien werden Organismen, die in der Lage sind diese Feinchemikalien herzustellen, kultiviert und die gewünschten Feinchemikalien aus den Organismen isoliert.

Für wirtschaftliche Verfahren zur biotechnologischen Herstellung von Feinchemikalien, aber auch für die Verwendung der Organismen als prozessierte oder nicht prozessierte Lebens- oder Futtermittel, ist es wünschenswert, den Gehalt an Feinchemikalien in den Organismen gezielt zu verändern, wie beispielsweise den Gehalt der gewünschten Feinchemikalie zu erhöhen und/oder den Metabolitfluß zu nicht gewünschten Feinchemikalien zu hemmen.

Wirtschaftlich bedeutende Feinchemikalien sind beispielsweise Plastochinone, Ubichinone sowie Verbindungen mit Vitamin E- oder Vitamin K-Aktivität, die eine Isoprenoid-Seitenkette aufweisen, die mit einem aromatischen Kern verbunden ist.

Die in der Natur vorkommenden acht Verbindungen mit Vitamin E-Aktivität sind Derivate des 6-Chromanols (Ullmann's Encyclopedia of Industrial Chemistry, Vol. A 27 (1996), VCH Verlagsgesellschaft, Chapter 4., 478-488, Vitamin E). Die Gruppe der Tocopherole (1a-d) weist eine gesättigte Seitenkette auf, die Gruppe der Tocotrienole (2a-d) eine ungesättigte Seitenkette: 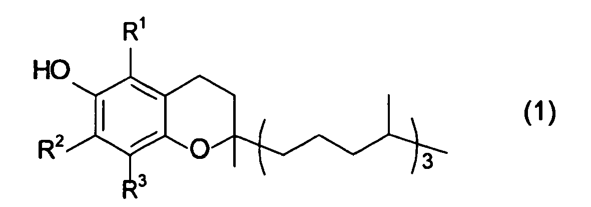 1a, α-Tocopherol: R¹ = R² = R³ = CH₃
1b, β-Tocopherol : R¹ = R³ = CH₃, R² = H
1c, γ-Tocopherol: R¹ = H, R² = R³ = CH₃
1d, δ-Tocopherol : R¹ = R² = H, R³ = CH₃ 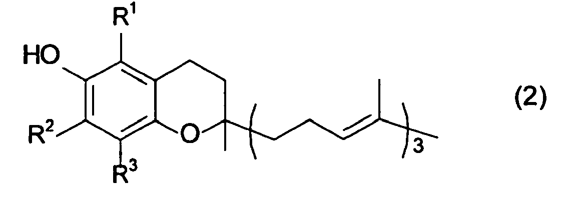 2a, α-Tocotrienol: R¹ = R² = R³ =CH₃
2b, β-Tocotrienol : R¹ = R³ = CH₃, R² = H
2c, γ-Tocotrienol: R¹ = H, R² = R³ = CH₃ ,
2d, δ-Tocotrienol: R¹ = R² = H, R³ = CH₃

In der vorliegenden Erfindung werden unter Vitamin E alle vorstehend erwähnten Tocopherole und Tocotrienole mit Vitamin-E-Aktivität verstanden.

Diese Verbindungen mit Vitamin-E-Aktivität sind wichtige natürliche fett-lösliche Antioxidantien. Ein Mangel an Vitamin E führt bei Menschen und Tieren zu pathophysiologischen Situationen. Vitamin E-Verbindungen haben daher einen hohen wirtschaftlichen Wert als Zusatzstoffe im Food- und Feed-Bereich, in pharmazeutischen Formulierungen und in kosmetischen Anwendungen.

Die in der Natur vorkommenden Verbindungen mit Vitamin K-Aktivität sind Derivate des 1,4-Naphthochinons (Ullmann's Encyclopedia of Industrial Chemistry, Vol. A 27 (1996), VCH Verlagsgesellschaft, Chapter 5., 488-506, Vitamin K).
Phyllochinon (frühere Bezeichnung: Vitamin K₁) weist eine größtenteils gesättigte Seitenkette auf, während die Gruppe der Menachinone-n (frühere Bezeichnung: Vitamin K₂) eine ungesättigte Seitenkette mit 4 bis 13 Isoprenylresten aufweist.

In der vorliegenden Erfindung werden unter Vitamin K alle Verbindungen mit Vitamin K-Aktivität verstanden, insbesondere die vorstehend erwähnten Verbindungen.

Ausgangspunkt der Biosynthese der Isoprenoidseitenkette ist Isopentenylpyrophosphat (IPP). IPP steht im Gleichgewicht mit seinem Isomer Dimethylallyl Pyrophosphat (DMAPP). Eine Kondensation von IPP mit DMAPP in Kopf-Schwanz Anlagerung ergibt das Monoterpen (C10) Geranyl-Pyrophosphat (GPP). Die Addition von weiteren IPP Einheiten führt zum Sesquiterpen (C15) Farnesy-Pyrophosphat (FPP) und zum Diterpen (C20) Geranyl-Geranyl-Pyrophosphat (GGPP).

Phyllochinon enthält eine C20 Phytyl-Kette, in der nur die erste Isopren-Einheit eine Doppelbindung enthält. GGPP wird durch die Geranylgeranyl-Pyrophosphat-Oxidoreduktase (GGPPOR) zum Phytyl-Pyrophosphat (PPP) umgeformt, dem Ausgangsstoff für die weitere Bildung von Tocopherolen.

Bei den Ringstrukturen der gemischten Prenyllipide, die zur Bildung der Vitamine E und K führen, handelt es sich um Chinone, deren Ausgangsmetabolite aus dem Shikimat-Weg stammen.

Chorismat wird ausgehend von Erythrose-4-Phosphat und Phosphoenolpyruvat (PEP) durch deren Kondensation zu 3-deoxy-D-Arabino-heptulosonat-7-Phosphat (DAHP) über die Zwischenstufen 3'-Dehydroquinat, 3'-Dehydroshikimat, Shikimat, Shikimat-3-Phosphat und 5'-Enolpyruvylshikimat-3-Phosphat gebildet. Dabei wird das Erythrose-4-Phosphat vom Calvinzyklus gebildet und das PEP von der Glykolyse bereitgestellt.

In höheren Pflanzen wird Tyrosin ausgehend von Chorismat über Prephenat und Arogenat gebildet. Die aromatische Aminosäure Tyrosin wird in Hydroxyphenyl-Pyruvat umgewandelt, welches durch Dioxygenierung in Homogentisinsäure überführt wird.

Die Homogentisinsäure wird anschließend an Phytylpyrophosphat (PPP) bzw. Geranylgeranylpyrophosphat gebunden, um die Vorläufer von α-Tocopherol und α-Tocotrienol, das 2-Methyl-6-phytylhydrochinon bzw. das 2-Methyl-6-geranylgeranylhydrochinon zu bilden. Durch Methylierungsschritte mit S-Adenosylmethionin als Methyl-Gruppen-Donor entsteht zunächst 2,3-Dimethyl-6-phytylquinol, dann durch Zyklisierung γ-Tocopherol und durch nochmalige Methylierung α-Tocopherol.

Es ist bekannt, durch Überexpression bzw. Herunterregulation von Biosynthesegenen des Tocopherolbiosyntheseweges, unter dem in der vorliegenden Erfindung der Biosyntheseweg von Hydroxyphenylpyruvat bis Tocopherol verstanden wird, den Gehalt an Vitamin E in Pflanzen zu modifizieren.

WO 97/27285 beschreibt eine Modifikation des Tocopherol-Gehaltes durch verstärkte Expression bzw. durch Herunterregulation des Enzyms p-Hydroxyphenylpyruvatdioxygenase (HPPD).

WO 99/04622 bzw. D. DellaPenna et al., Science 1998, 282, 2098-2100 beschreiben Gensequenzen codierend für eine γ-Tocopherolmethyltransferase aus Synechocystis PCC6803 und Arabidopsis thaliana und deren Einbau in transgene Pflanzen, die einen modifizierten Vitamin E-Gehalt aufweisen.

Ferner ist bekannt, durch Überexpression bzw. Herunterregulation von Biosynthesegenen des Biosyntheseweges der Isoprenoid-Seitenkette, den Gehalt an Vitamin E in Pflanzen zu modifizieren.

WO 99/23231 zeigt, daß die Expression einer Geranylgeranyl-Reductase in transgenen Pflanzen eine gesteigerte Tocopherolbiosynthese zur Folge hat.

WO 00/08169 beschreibt Gensequenzen codierend eine 1-Deoxy-D-Xylose-5-Phosphat-Synthase und eine Geranyl-Geranyl-Pyrophosphat Oxidoreduktase un deren Einbau in transgene Pflanzen, die einen modifizierten Vitamin E-Gehalt aufweisen.

Alle diese Methoden liefern zwar Organismen, insbesondere Pflanzen, die einen modifizierten Gehalt an der Feinchemikalie Vitamin E aufweisen, dennoch ist oft die Höhe des Gehalts an Vitamin E für Verfahren zur Herstellung von Vitamin E durch Isolierung aus diesen transgenen Organismen noch nicht zufriedenstellend.

Der Erfindung lag daher die Aufgabe zugrunde ein weiteres Verfahren zur Herstellung von Feinchemikalien durch Kultivieren von Organismen, bzw. transgene Organismen die Feinchemikalien herstellen können, mit optimierten Eigenschaften zur Verfügung zu stellen, die die geschilderten Nachteile des Standes der Technik nicht aufweisen.

Demgemäß wurde ein Verfahren zur Herstellung von Vitamin E gefunden, indem man Organismen kultiviert, die gegenüber dem Wildtyp einen genetisch veränderten Shikimatweg aufweisen.

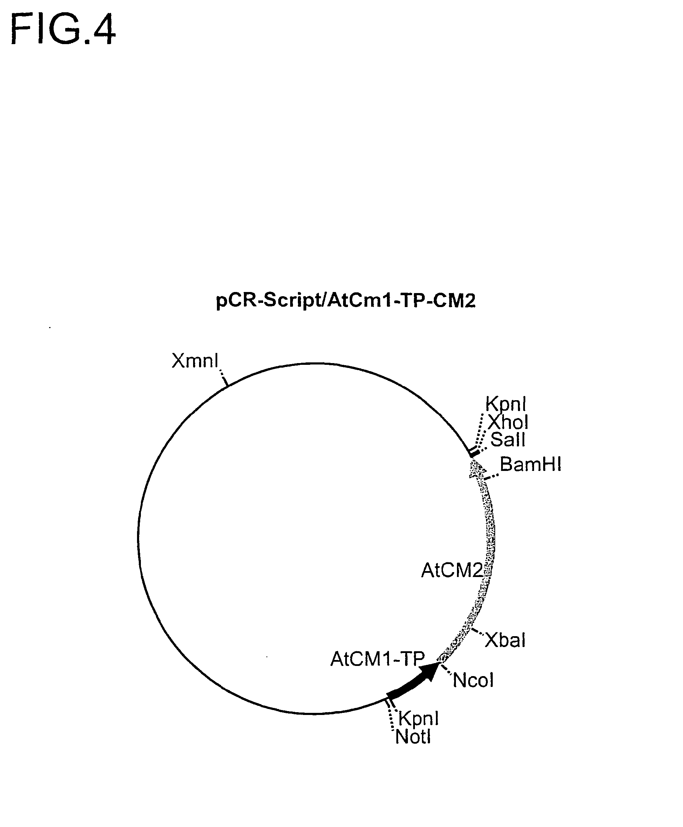

Unter Shikimatweg wird in der vorliegenden Erfindung, insbesondere für höhere Pflanzen der vorstehend beschriebene Biosyntheseweg ausgehend von D-Erythrose-4-Phosphat über Shikimat, Chorismat, Prephenat, Arogenat, Tyrosin bis einschließlich zu 4-Hydroxyphenylpyruvat verstanden (G. Michal, Biochemical Pathways, Biochemie-Atlas, Spektrum Akademischer Verlag Heidelberg, Berlin, 1999, Seite 59 bis 60, Abb. 4.7-1 und Kapitel 4.7.1)

Vorzugsweise wird in der vorliegenden Erfindung unter Shikimatweg der Stoffwechselweg von Shikimat bis 4-Hydroxyphenylpyruvat, besonders bevorzugt der Stoffwechselweg von Chorismat bis 4-Hydroxyphenylpyruvat verstanden, wobei für Pflanzen der Stoffwechselweg ab Chorismat über Prephenat, Arogenat und Tyrosin verläuft.

Unter Feinchemikalien werden Stoffwechselprodukte des Organismus verstanden, die aus dem Shikimatweg resultieren. Der Shikimatweg beginnt dabei bei D-Erythrose-4-Phosphat und endet bei 4-Hydroxyphenylpyruvat, wie vorstehend beschrieben. Für diese Stoffwechselprodukte stellen die Ausgangsverbindung D-Erythrose-4-Phosphat, die Endverbindung 4-Hydroxyphenylpyruvat sowie alle, vorstehend erwähnten, Zwischenstufen des Shikimatweges, die Ausgangsverbindungen, nachstehend auch Zwischenverbindungen bezeichnet, dar, die biosynthetisch vom Organismus in die Stoffwechselprodukte umgewandelt werden.

Bevorzugte Feinchemikalien sind die aromatischen Aminosäuren, wie beispielsweise Phenylalanin, Tyrosin und Tryptophan, Salicylsäurederivate, Folsäurederivate, Phenylpropanoide, wie beispielsweise Lignin, Lignane oder Coumarine, insbesondere Scopoletin oder Scopolin, Flavonoide, wie beispielsweise Chalcone, Flavanone, Flavanole, Anthocyanidine oder Isoflavonoide, Stilbene, Xanthone, oder Chinonderivate, wie beispielsweise Vitamin E, Vitamin K, Ubichinone, Plastochinone oder Shikonin.

Besonders bevorzugte Feinchemikalien sind Vitamin E, Vitamin K oder Ubichinon, insbesondere Vitamin E.

Je nachdem ob die genetische Veränderung des Shikimatweges zu einer Erhöhung oder Erniedrigung des Metabolitflusses zu einer bestimmten Zwischenverbindung - die Teil des Shikimatweges ist - führt, erhöht sich bzw. erniedrigt sich der Gehalt der Feinchemikalie die biosynthetisch im Organismus aus dieser Zwischenverbindung hergestellt wird. Unter genetischer Veränderung des Shikimatweges wird somit vorzugsweise die Erhöhung oder Erniedrigung des Metabolitflußes zu einer Zwischenverbindung des Shikimatweges verstanden.

Genetische Veränderungen des Shikimatweges, die zu einer Erhöhung des Metabolitflusses zu einer Zwischenverbindung und damit der entsprechenden Feinchemikalie führen, sind beispielsweise die folgenden Maßnahmen A, B oder C:
A: Erhöhung der Aktivität mindestens eines Enzyms des Shikimatweges des Wildtyps,
   beispielsweise durch Überexpression von Genen des Shikimatweges, die Proteine mit dieser enzymatischen Aktivität codieren, durch das Ausschalten von negativen Regulationsmechanismen von zur Zwischenverbindung führenden Stoffwechselwegen, wie beispielsweise das Ausschalten der Feed-back-Inhibierung oder das Einbringen von orthologen Genen die im gewünschten Organismus keiner Regulation unterliegen.
B: Einbringen mindestens eines Gens in den Organismus zu dem der Wildtyp kein orthologes Gen aufweist und das den Stoffwechselweg des Shikimatweges des Wildtyps überbrückt. Dieses Gen kann beispielsweise durch die neue Genfunktion eine Erhöhung des Stoffflusses zu der Zwischenverbindung bewirken, bei der die Überbrückung endet.
C: Inaktivierung von Genen die Enzyme kodieren, die mit den Enzymen des Stoffwechselwegs, der zum gewünschten Produkt führt, konkurrieren.
   Genetische Veränderung des Shikimatweges die zu einer Erniedrigung des Metabolitflusses zu einer Zwischenverbindung und damit der entsprechenden Feinchemikalie führen, sind beispielsweise die folgenden Maßnahmen D, E oder F.
D: Überexpression eines Stoffwechselgens und damit die Erhöhung der entsprechenden Enzymaktivität, die von dieser Zwischenverbindung wegführt;
E: Inaktivierung von Genen die Enzyme kodieren, die zu dieser Zwischenverbindung führen, beispielsweise durch Antisense-Technik oder Kosuppression;
F: Expression eines Gens zu dem der Wildtyp kein orthologes Gen aufweist. Dieses Gen kann beispielsweise den Stoffwechselweg des Shikimatweges des Wildtyps überbrücken und durch die neue Genfunktion eine Erniedrigung des Stofflusses zu den überbrückten Zwischenverbindungen bewirken.

In dem erfindungsgemäßen Verfahrens führt die genetische Veränderung des Shikimatweges im Organismus zu einer Erhöhung des Metabolitflusses zu einer gewünschten Zwischenverbindung und damit der entsprechenden gewünschten Feinchemikalie.

Die Erhöhung des Metabolitflusses zu einer gewünschten Zwischenverbindung des Shikimatweges und damit zur gewünschten Feinchemikalie erfolgt durch mindestens eine Maßnahme ausgewählt aus der Gruppe der Maßnahme A und B, also durch die Maßnahme A und/oder B, wobei die Maßnahmen A und B die vorstehend beschriebene Bedeutung haben.

Das erfindungsgemäße Verfahren ist daher dadurch gekennzeichnet, daß man zur genetischen Veränderung des Shikimatweges mindestens eine Maßnahme ausgewählt aus der Gruppe der Maßnahmen A und B durchführt, wobei A und B folgende Bedeutung haben:
A: Erhöhung der Aktivität mindestens eines Enzyms des Shikimatweges des Wildtyps;
B: Einbringen mindestens eines Gens in den Organismus zu dem der Wildtyp kein orthologes Gen aufweist und das den Stoffwechselweg des Shikimatweges des Wildtyps überbrückt.

Die Erhöhung der Aktivität mindestens eines Enzyms des Shikimatweges des Wildtyps nach Maßnahme A kann beispielsweise durch Überexpression von Nukleinsäuren, also Genen des Shikimatweges, die Proteine mit dieser enzymatischen Aktivität codieren, durch das Ausschalten von negativen Regulationsmechanismen von zur Zwischenverbindung führenden Stoffwechselwegen, wie beispielsweise das Ausschalten der Feed-back-Inhibierung oder das Einbringen von orthologen Genen erfolgen, die im gewünschten Organismus keiner Regulation unterliegen.

Vorzugsweise erfolgt die Erhöhung der Aktivität mindestens eines Enzyms des Shikimatweges des Wildtyps gemäß Maßnahme A durch Überexpression von Nukleinsäuren des Shikimatweges, die Proteine mit dieser enzymatischen Aktivität codieren.

In dem erfindungsgemäßen Verfahren erfolgt die Durchführung der Maßnahme A dadurch, daß man eine Nukleinsäure, codierend eine Chorismatmutase, in den Organismus einbringt.

Unter einer Chorismatmutase wird ein Protein verstanden, das die enzymatische Aktivität aufweist, Chorismat in Prephenat umzuwandeln.

Prinzipiell sind alle Chorismatmutasen im erfindungsgemäßen Verfahren verwendbar, wie beispielsweise die Chorismatmutase aus *Petroselinum Crispum* (Accessionsnummer: T14902, T14901), Chorismatmutase aus Streptomyces coelicolor (T36865), Chorismatmutase aus Bacillus subtilis (A33894), Chorismatmutase aus Aspergillus nidulans (AAD30065) oder die nachstehend beschriebenen Chorismatmutasen aus Arabidopsis thaliana oder die nachstehend beschriebene Chorismatmutase Aktivität der Chorismatmutase-Prephenatdehydrogenase (tyrA) aus E. coli.

In einer bevorzugten Ausführungsform werden Chorismatmutasegene verwendet, die eine Chorismatmutase kodieren, deren Aktivität einer reduzierten posttranslationalen Regulation im Organismus unterliegt. Unter einer reduzierten Regulation wird eine Regulation der Aktivität von höchstens 99 %, vorzugsweise höchstens 70 %, besonders bevorzugt 50 %, insbesondere bevorzugt 0 %, also keine Regulation der Aktivität, verglichen mit der Wildtypregulation verstanden.

Chorismatmutasegene, die eine Chorismatmutase kodieren, deren Aktivität im Organismus einer reduzierten, insbesondere keiner Regulation unterliegt, sind beispielsweise Chorismatmutasegene aus gattungsverschiedenen Organismen oder Chorismatmutasegene aus dem gleichen Organismus oder gattungsverwandten Organismen die an der Lokalisation der Expression einer reduzierten, insbesondere keiner posttranslationalen Regulation unterliegen.

Unter Organismen werden erfindungsgemäß prokaryontische Organismen oder eukaryontische Organismen, wie beispielsweise Bakterien, Hefen, Algen, Moose, Pilze oder Pflanzen, verstanden, die in der Lage sind, als Wildtyp oder durch genetische Veränderung die vorstehend erwähnten Feinchemikalien herzustellen. Bevorzugte Organismen sind photosynthetisch aktive Organismen, wie beispielsweise Cyanobakterien, Moose, Algen oder Pflanzen, die bereits als Wildtyp in der Lage sind, die vorstehend erwähnten Feinchemikalien herzustellen.

Besonders bevorzugte Organismen sind Pflanzen.

In einer weiter bevorzugten Ausführungsform der Maßnahme A des erfindungsgemäßen Verfahrens werden Chorismatmutasegene, die eine Chorismatmutase kodieren, deren Aktivität in Pflanzen einer reduzierten posttranslationalen Regulation unterliegt, in Pflanzen eingebracht.

Beispielsweise sind dies einige bakterielle oder davon abgeleitete Chorismatmutasegene, also Nukleinsäuren, die ein Protein kodieren, enthaltend die Aminosäuresequenz einer bakteriellen Chorismatmutase deren Aktivität in Pflanzen einer reduzierten posttranslationalen Aktivität unterliegt, beispielsweise die nachstehend beschriebene Nukleinsäure kodierend für die Chorismatmutase Aktivität der Chorismatmutase-Prephenatdehydrogenase (tyrA) aus E. coli. oder eine von dieser Sequenz durch Substitution, Insertion oder Deletion von Aminosäuren abgeleitete Sequenz, die eine Homologie von mindestens 30 %, vorzugsweise mindestens 50 %, bevorzugter mindestens 70 %, besonders bevorzugt mindestens 90 % auf Aminosäureebene mit der Sequenz der bakteriellen Chorismatmutase und die enzymatische Eigenschaft einer Chorismatmutase aufweist.

Unter dem Begriff "Substitution" ist in der Beschreibung der Austausch einer oder mehrerer Aminosäuren durch eine oder mehrere Aminosäuren zu verstehen. Bevorzugt werden sog. konservative Austausche durchgeführt, bei denen die ersetzte Aminosäure eine ähnliche Eigenschaft hat wie die ursprüngliche Aminosäure, beispielsweise Austausch von Glu durch Asp, GIn durch Asn, Val durch Ile, Leu durch Ile, Ser durch Thr.

Deletion ist das Ersetzen einer Aminosäure durch eine direkte Bindung. Bevorzugte Positionen für Deletionen sind die Termini des Polypeptides und die Verknüpfungen zwischen den einzelnen Proteindomänen.

Insertionen sind Einfügungen von Aminosäuren in die Polypeptidkette, wobei formal eine direkte Bindung durch ein oder mehrere Aminosäuren ersetzt wird.

Unter Homologie zwischen zwei Proteinen wird vorzugsweise die Identität der Aminosäuren über die jeweils gesamte Proteinlänge verstanden, vorzugsweise die Identität die durch Vergleich mit Hilfe des Programmalgorithmus GAP (UWGCG, University of Wisconsin, Genetic Computer Group) unter Einstellung folgender Parameter berechnet wird:

| | |
|---|---|
| Gap Weight: | 12 |
| Length Weight: | 4 |
| Average Match: | 2,912 |
| Average Mismatch: | -2,003 |

Unter einem Protein, das eine Homologie von mindestens 30 % auf Aminosäureebene mit der Sequenz der vorstehend beschriebenen Chorismatmutase aus E. coli aufweist, wird dementsprechend ein Protein verstanden, das bei einem Vergleich seiner Sequenz mit der Sequenz der vorstehend beschriebenen Chorismatmutase, vorzugsweise nach obigen Programmalgorithmus mit obigem Parametersatz eine Homologie von mindestens 30 % aufweist.

Die bakteriellen oder davon abgeleiteten Chorismatmutasegene können auch Proteine kodieren die die Eigenschaft einer Chorismatmutase und die Eigenschaft eines weiteren Enzyms aufweisen, wie beispielsweise das nachstehend beschriebene Chorismatmutase-Prephenatdehydrogenase-Gen (tyrA) aus E.coli K12. Diese Ausführungsform ist, wie nachstehend beschrieben, besonders bevorzugt, wenn die Maßnahmen A und B in Kombination durchgeführt werden.

In einer besonders bevorzugten Ausführungsform der Maßnahme A des erfindungsgemäßen Verfahren, insbesondere bei der Durchführung der Maßnahme A alleine, werden die Chorismatmutasegene in spezifische Orte-in den Organismus gebracht, an denen die entsprechenden Chorismatmutasen einer reduzierten posttranslationalen Regulation unterliegen.

Vorzugsweise werden dabei Nukleinsäuren, kodierend eine Chorismatmutase aus dem gleichen oder aus gattungsverwandten Organismen verwendet, die am Ort der Expression einer reduzierten posttranslationalen Regulation unterliegen.

Die Isoformen von Chorismatmutasen die aus unterschiedlichen Kompartimenten eines Organismus isoliert werden, weisen eine unterschiedliche Regulation auf.

Die entsprechenden Corismatmutasegene aus einem spezifischen Kompartiment des Organismus oder aus gattungsverwandten Organismen können in andere Kompartimente des Organismus eingebracht werden, in denen die kodierten Chorismatmutasen keiner posttranslationalen Regulation unterliegen.

In einer besonders bevorzugten Ausführungsform des erfindungsgemäßen Verfahrens in Pflanzen wird zur Durchführung der Maßnahme A eine Nukleinsäure, codierend eine cytosolische Chorimatmutase aus Pflanzen in Plastiden von Pflanzen eingebracht.

Prinzipiell eignen sich dafür alle Nukleinsäuren, die eine cytosolische Chorismatmutase aus Pflanzen kodieren, vorzugsweise die Nukleinsäure kodierend eine cytosolische Chorismatmutase aus Arabidopsis thaliana (Seq ID NO. 3) und davon abgeleitete natürliche oder nicht natürliche Nukleinsäuren.

Die Existenz verschiedener Isoformen der Chorismatmutase konnte für verschiedene Organismen nachgewiesen werden. So konnten aus Arabidopsis thaliana drei verschiedene Chorismatmutasen isoliert werden (Eberhard et al.1993. FEBS 334, 233-236; Eberhard et al. 1996. Plant J. 10, 815-821; Mobley et a1.1999.Gene 15;240(1):115-123).

Diese Isoformen unterschieden sich in ihrer Lokalisation als auch in ihren enzymatischen Eigenschaften. So ist die Chorismatmutase-1 plastidär lokalisiert und wird durch die aromatischen Aminosäuren allosterisch kontrolliert.

Das cytosolische Isoenzym Chorismatmutase-2 unterliegt keiner bekannten Regulation (Benesova, M. Bode, R, Phytochemistry 1992, 31, 2983-2987).

Unter Nukleinsäuren, kodierend eine cytosolische Chorismatmutase aus Arabidopsis thaliana und.davon abgeleitete natürliche oder nicht natürliche Nukleinsäuren werden Nukleinsäuren verstanden, die ein Protein kodieren, enthaltend die Aminosäuresequenz der cytosolischen Chorismatmutase (SEQ ID NO. 4) oder eine von dieser Sequenz durch Substitution, Insertion oder Deletion von Aminosäuren abgeleitete Sequenz, die eine Homologie von mindestens 30 %, vorzugsweise mindestens 50 %, bevorzugter mindestens 70 %, besonders bevorzugt mindestens 90 % auf Aminosäureebene mit der Sequenz SEQ ID NO. 4 und die enzymatische Eigenschaft einer Chorismatmutase aufweisen.

Unter einem Protein, das eine Homologie von mindestens 30 % auf Aminosäureebene mit der Sequenz SEQ ID NO. 4 aufweist, wird dementsprechend ein Protein verstanden, das bei einem Vergleich seiner Sequenz mit der Sequenz SEQ ID NO. 4, vorzugsweise nach vorstehendem Programmalgorithmus mit vorstehenden Parametersatz eine Homologie von mindestens 30 % aufweist.

In einer weiter bevorzugten Ausführungsform des erfindungsgemäßen Verfahrens wird eine Nukleinsäure, codierend die cytosolische Chorismatmutase aus Arabidopsis thaliana (SEQ ID NO. 4) in Plastiden von Pflanzen eingebracht.

Geeignete Nukleinsäuresequenzen sind beispielsweise durch Rückübersetzung der Polypeptidsequenz gemäß dem genetischen Code erhältlich.

Bevorzugt werden dafür solche Codons verwendet; die entsprechend der pflanzenspezifischen codon usage häufig verwendet werden. Die codon usage läßt sich anhand von Computerauswertungen anderer, bekannter Gene der betreffenden Pflanze leicht ermitteln.

In einer weiter besonders bevorzugten Ausführungsform des erfindungsgemäßen Verfahrens wird eine Nukleinsäure der Sequenz SEQ ID NO. 3 in Plastiden von Pflanzen eingebracht. Die Sequenz SEQ ID NO. 3 stellt das Gen der cytosolischen Chorismatmutase aus Arabidopsis thaliana (Chorismatmutase-2) dar.

Das Einbringen der Nukleinsäuren, codierend eine Chorismatmutase in Plastiden von Pflanzen kann beispielsweise, wie nachstehend für die Chorismatmutase-Prephenatdehydrogenase näher beschrieben, durch Einbringen von Expressionskassetten in Pflanzen erreicht werden, deren Nukleinsäure-Sequenz für ein Chorismatmutase-Fusionsprotein kodiert, wobei ein Teil des Fusionsproteins ein Transitpeptid ist, das die Translokation des Polypeptides steuert. Bevorzugt sind für die Chloroplasten spezifische Transitpeptide, welche nach Translokation der cytosolischen Chorismatmutase in die Chloroplasten vom Chorismatmutase-Teil enzymatisch abgespalten werden.

In einer weiteren besonders bevorzugten Ausführungsform des erfindungsgemäßen Verfahrens bringt man daher ein Nukleinsäurekonstrukt in die Pflanze ein, enthaltend eine Nukleinsäure kodierend ein plastidäres Transitpeptid und eine Nukleinsäure die ein Protein kodiert, enthaltend die Aminosäuresequenz SEQ ID NO. 4 oder eine von dieser Sequenz durch Substitution, Insertion oder Deletion von Aminosäuren abgeleitete Sequenz, die eine Homologie von mindestens 30% auf Aminosäureebene mit der Sequenz SEQ ID NO. 4 und die enzymatische Eigenschaft einer Chorismatmutase aufweist.

Nukleinsäuren, codierend plastidäre Transitpeptide sind beispielsweise DNA-Sequenzen von drei Kassetten des Plastiden-Transitpeptids der plastidären Transketolase aus Tabak in drei Leserastern als KpnI/BamHI Fragmente mit einem ATG-Codon in der NcoI Schnittstelle:
pTP09
pTP10
pTP11 oder die Nukleinsäure, codierend das plastidäre Transitpeptid der plastidären Chorismatmutase-1 aus Arabidopsis thaliana (SEQ ID NO. 7): Vorzugsweise wird zur plastidären Lokalisation einer cytosolischen Chorismatmutase die Nukleinsäure, codierend das plastidäre Transitpeptid der plastidären Chorismatmutase-1 aus Arabidopsis thaliana verwendet.

In einer weiteren besonders bevorzugten Ausführungsform des erfindungsgemäßen Verfahrens bringt man daher ein Nukleinsäurekonstrukt in die Pflanze ein, enthaltend eine Nukleinsäure kodierend ein plastidäres Transitpeptid der plastidären Chorismatmutase-1 aus Arabidopsis thaliana und eine Nukleinsäure die ein Protein kodiert, enthaltend die Aminosäuresequenz SEQ ID NO. 4 oder eine von dieser Sequenz durch Substitution, Insertion oder Deletion von Aminosäuren abgeleitete Sequenz, die eine Homologie von mindestens 30 % auf Aminosäureebene mit der Sequenz SEQ ID NO. 4 und die enzymatische Eigenschaft einer Chorismatmutase aufweist.

Besonders bevorzugt wird für Maßnahme A des erfindungsgemäßen Verfahrens ein Nukleinsäurekonstrukt enthaltend die Sequenz (SEQ ID NO. 5) in Pflanzen eingebracht.

SEQ ID NO. 5 stellt ein Nukleinsäurekonstrukt aus der Nukleinsäure, codierend das plastidäre Transitpeptid der plastidären Chorismatmutase-1 aus Arabidopsis thaliana und der Nukleinsäure, codierend die cytosolische Chorismatmutase-2 aus Arabidopsis thaliana dar.

Die vorliegende Anmeldung betrifft insbesondere auch die Verwendung dieser Nukleinsäurekonstrukte in der Maßnahme A des erfindungsgemäßen Verfahrens.

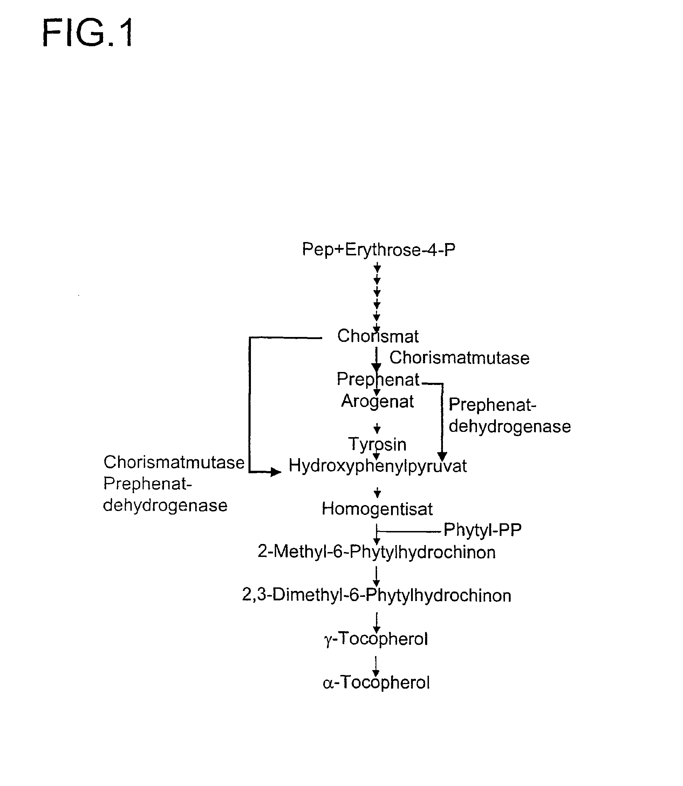

Figur 1 zeigt beispielsweise das Biosyntheseschema ausgehend von Erythrose-4-Phosphat zu Vitamin E. Durch die zusätzliche Expression eines Chorismatmutasegens wird der Shikimatweg des Wildtyps genetisch verändert und der Metabolitfluß zu Hydroxyphenylpyruvat erhöht. Das nun vermehrt zur Verfügung stehende Hydroxyphenylpyruvat wird weiter in Richtung Tocopherole umgesetzt. Ein erhöhter Gehalt an Hydroxyphenylpyruvat führt zu einer erhöhten Umsetzung Richtung Vitamin E und/oder Vitamin K. Vorzugsweise führt ein erhöhter Gehalt an Hydroxyphenylpyruvat zu-einer Erhöhung des Vitamin E-Gehalts.

Die Herstellung einer Expressionskassette erfolgt, wie nachstehend ausführlich beschrieben, durch Fusion eines geeigneten Promotors mit einer geeigneten Chorismatmutase-Nukleinsäure-Sequenz und vorzugsweise einer zwischen Promotor und Chorismatmutase-Nukleinsäure-Sequenz inserierten Nukleinsäure, die für ein plastidäres Transitpeptid kodiert, also vorzugsweise durch Fusion eines geeigneten Promotors mit einem geeigneten, vorstehend beschriebenen Nukleinsäurekonstrukt, sowie einem Polyadenylierungssignal nach gängigen Rekombinations- und Klonierungstechniken, wie sie beispielsweise in T. Maniatis, E. F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley-Interscience (1987) beschrieben sind.

Die Maßnahme B zur Veränderung des Shikimatweges des Wildtyps erfolgt, wie vorstehend ausgeführt durch Einbringen mindestens eines Gens in den Organismus zu dem der Wildtyp kein orthologes Gen aufweist und das den Stoffwechselweg des Shikimatweges des Wildtyps überbrückt. Dieses Gen codiert ein Enzym, daß durch die neue enzymatische Aktivität eine Erhöhung des Stoffflusses zu der Zwischenverbindung bewirkt, bei der die Überbrückung endet. Diese neue enzymatische Aktivität unterliegt vorzugsweise keiner Regulation durch den Organismus, schließt also den Stoffwechselweg kurz, um beispielsweise limitierende Regulationsstellen im Stoffwechsel zu umgehen. Dadurch ist es möglich, den Metabolitfluß zu limitierenden Substanzen von vorhandenen Regulationen zu entkoppeln.

Unter einem zum Wildtyp orthologen Gen wird ein Gen verstanden, das aus einem anderen Organismus stammt, wobei die Enzymaktivität die das Gen kodiert bereits im Wildtyp vorhanden ist.

Dementsprechend wird unter der Formulierung "Gen, zu dem der Wildtyp kein orthologes Gen aufweist" ein Gen aus einem anderen Organismus verstanden, wobei die Enzymaktivität die das Gen kodiert vor der Transformation im Wildtyp nicht vorhanden oder nicht aktiviert war.

Vorzugsweise wir unter einem zum Wildtyp orthologen Gen ein funktionelles Äquivalent aus einem anderen Organismus verstanden, wobei unter funktionellem Äquivalent die Gesamtheit der Eigenschaften des Genproduktes (Protein) verstanden wird.

Dementsprechend wird vorzugsweise unter der Formulierung "Gen, zu dem der Wildtyp kein orthologes Gen aufweist" ein Gen verstanden, zu dem der Wildtyp kein funktionelles Äquivalent gemäß der vorstehend gegebenen Definition besitzt und somit eine Stoffwechselleistung etabliert wird, die einen alternativen Stoffwechselweg erzeugt, um ein bereits in der Pflanze vorhandenes Produkt (enthaltenen Metaboliten) zu produzieren.

Unter Organismen werden erfindungsgemäß, wie vorstehend für Maßnahme A beschrieben, prokaryontische Organismen oder eukaryontische Organismen, wie beispielsweise Bakterien, Hefen, Algen, Moose, Pilze oder Pflanzen, verstanden, die in der Lage sind, als Wildtyp oder durch genetische Veränderung die vorstehend erwähnten Feinchemikalien herzustellen. Bevorzugte Organismen sind photosynthetisch aktive Organismen, wie beispielsweise Cyanobakterien, Moose, Algen oder Pflanzen, die bereits als Wildtyp in der Lage sind, die vorstehend erwähnten Feinchemikalien herzustellen.

Besonders bevorzugte Organismen sind Pflanzen.

In einer besonders bevorzugten Ausführungsform des erfindungsgemäßen Verfahrens verwendet man deshalb Pflanzen als zu transformierende Organismen. In diesem Fall eignen sich für die Durchführung der Maßnahme B vorzugsweise bakterielle Gene als Gene zu denen die Pflanze kein orthologes Gen aufweist.
In einer bevorzugten Ausführungsform der Maßnahme B des erfindungsgemäßen Verfahrens wird der Stoffwechselweg des Shikimatweges der Pflanze durch das mindestens eine eingebrachte Gen überbrückt.

In einer besonders bevorzugten Ausführungsform der Maßnahme B des erfindungsgemäßen Verfahrens bringt man eine Nukleinsäure, codierend eine Prephenatdehydrogenase in eine Pflanze ein. Für die bevorzugte Ausführungsform des erfindungsgemäßen Verfahrens eignen sich alle Gene die eine Prephenatdehydrogenase kodieren.

Unter einer Prephenatdehydrogenase wird ein Enzym verstanden, das die enzymatische Aktivität aufweist, Prephenat in 4-Hydroxyphenylpyruvat umzuwandeln.

Beispiele für Nukleinsäuren, die eine Prephenatdehydrogenase codieren und im erfindungsgemäßen Verfahren verwendet werden können sind die bekannten und beispielsweise in Datenbanken im Internet zugänglichen Prephenatdehydrogenasegene aus Lactococcus lactis (Accession X78413), Synechocystis spec PCC 6803 (slr2081), Deinococcus radiodurans (AAF10695) oder Bacillus subtilis (P20692). Weitere Beispiele können durch Vergleich der Homologien der Sequenzen mit diesen bekannten Prephenatdehydrogenasegenen gefunden werden, wie beispielsweise die potentiellen Prephenatdehydrogenase aus Termotoga maitima (AAD35430) oder Heliobacter pylori 26695 (Accession AAD08422).

In einer bevorzugten Ausführungsform des erfindungsgemäßen Verfahrens unter Verwendung eines Prephenatdehydrogenasegens bringt man eine Nukleinsäure ein, die ein Protein kodiert, enthaltend die Aminosäuresequenz der Prephenatdehydrogenase aus Synechocystis spec PCC 6803 oder eine von dieser Sequenz durch Substitution, Insertion oder Deletion von Aminosäuren abgeleitete Sequenz, die eine Homologie von mindestens 30 %, vorzugsweise mindestens 50 %, bevorzugter mindestens 70 %, besonders bevorzugt mindestens 90 % auf Aminosäureebene mit der Sequenz der Prephenatdehydrogenase aus Synechocystis spec PCC 6803 und die enzymatische Eigenschaft Prephenatdehydrogenase aufweist.

Unter einem Protein, das eine Homologie von mindestens 30 % auf Aminosäureebene mit der Sequenz der Prephenatdehydrogenase aus Synechocystis spec PCC 6803 aufweist, wird dementsprechend ein Protein verstanden, das bei einem Vergleich seiner Sequenz mit der Sequenz der Prephenatdehydrogenase aus Synechocystis spec PCC 6803, vorzugsweise nach vorstehendem Programmalgorithmus mit vorstehendem Parametersatz eine Homologie von mindestens 30 % aufweist. Figur 1 zeigt beispielsweise das Biosyntheseschema ausgehend von Erythrose-4-Phosphat zu den Tocopherolen. Durch die zusätzliche Expression eines Prephenatdehydrogenase-Gens wird der Shikimatweg des Wildtyps genetisch verändert und der Metabolitfluß zu Hydroxyphenylpyruvat erhöht. Das nun vermehrt zur Verfügung stehende Hydroxyphenylpyruvat wird weiter in Richtung Tocopherole umgesetzt. Ein erhöhter Gehalt an Hydroxyphenylpyruvat führt zu einer erhöhten Umsetzung Richtung Vitamin E und/oder Vitamin K. Vorzugsweise führt ein erhöhter Gehalt an Hydroxyphenylpyruvat zu einer Erhöhung des Vitamin E-Gehalts.

Es ist jedoch vorteilhaft, gegebenenfalls in Kombination mit der erfindungsgemäßen Überbrückung des Stoffwechselweges weitere Enzyme des Shikimatweges überzuexprimieren, um einen erhöhten Metabolitfluß zu den gewünschten Feinchemikalien zu erreichen.

In einer weiteren, bevorzugten Ausführungsform des erfindungsgemäßen Verfahrens werden daher die Maßnahmen A und B in Kombination durchgeführt.

In einer besonders bevorzugten Ausführungsform dieser erfindungsgemäßen Verfahrensvariante bringt man eine Nukleinsäure codierend eine Prephenatdehydrogenase in Kombination mit einer Nukleinsäure codierend eine Chorismatmutase in eine Pflanze ein.

Diese Kombination kann beispielsweise durch Einbringen von zwei Nukleinsäuren erfolgen, die jeweils ein Enzym mit der Aktivität einer Chorismatmutase und ein Enzym mit der Aktivität einer Prephenatdehydrogenase codieren. Für diese Ausführungsform ist es notwendig, zwei verschiedene Nukleinsäuren, die jeweils eines dieser Enzyme kodieren, in die Pflanze einzubringen.

In einer insbesondere bevorzugten Ausführungsform des erfindungsgemäßen Verfahrens erfolgt diese Kombination in einer Nukleinsäure, in dem man eine Nukleinsäure, codierend eine Chorismatmutase-Prephenatdehydrogenase in eine Pflanze einbringt.

Das Chorismatmutase-Prephenatdehydrogenase-Gen, codiert ein Protein, das sowohl die enzymatischen Eigenschaften einer Chorismatmutase als auch einer Prephenatdehydrogenase aufweist. Dadurch wird durch Einbringen einer Nukleinsäure eine enzymatische Aktivität überexprimiert, bzw. eine enzymatische Aktivität eingebracht, die einer reduzierten posttranslationalen Regulation unterliegt (Chorismatmutase) und eine enzymatische Eigenschaft (Prephenatdehydrogenase) neu eingeführt.

Unter einer-Chorismatmutase-Prephenatdehydrogenase wird ein Enzym verstanden, das die enzymatische Aktivität aufweist, Chorismat in 4-Hydroxyphenylpyruvat umzuwandeln.

In einer weiter besonders bevorzugten Ausführungsform dieser erfindungsgemäßen Verfahrensvariante bringt man eine Nukleinsäure ein, die ein Protein kodiert, enthaltend die Aminosäuresequenz SEQ ID NO. 2 oder eine von dieser Sequenz durch Substitution, Insertion oder Deletion von Aminosäuren abgeleitete Sequenz, die eine Homologie von mindestens 30 %, vorzugsweise mindestens 50 %, bevorzugter mindestens 70 %, besonders bevorzugt mindestens 90 % auf Aminosäureebene mit der Sequenz SEQ ID NO. 2 und die enzymatische Eigenschaft einer Chorismatmutase-Prephenatdehydrogenase aufweist.

Das Protein mit der Aminosäuresequenz SEQ ID NO. 2 stellt die Chorismatmutase-Prephenatedehydrogenase (*tyrA*) aus *E.coli* K12 dar.

Unter einem Protein, das eine Homologie von mindestens 30 % auf Aminosäureebene mit der Sequenz SEQ ID NO.2 aufweist, wird dementsprechend ein Protein verstanden, das bei einem Vergleich seiner Sequenz mit der Sequenz SEQ ID NO.2, vorzugsweise nach vorstehendem Programmalgorithmus mit vorstehendem Parametersatz eine Homologie von mindestens 30 % aufweist.

Alle in der Beschreibung erwähnten Nukleinsäuren können beispielsweise eine RNA-, DNA- oder cDNA-Sequenz sein.

Geeignete Nukleinsäuresequenzen sind, wie vorstehend beschrieben, durch Rückübersetzung der Polypeptidsequenz gemäß dem genetischen Code erhältlich.

Bevorzugt werden dafür solche Codons verwendet, die entsprechend der Organismus spezifischen codon usage häufig verwendet werden. Die codon usage läßt sich anhand von Computerauswertungen anderer, bekannter Gene des betreffenden Organismus leicht ermitteln.

Soll das Protein beispielsweise in einer Pflanze exprimiert werden, so ist es häufig vorteilhaft, die codon usage der Pflanze bei der Rückübersetzung zu verwenden.

Weitere bevorzugte Chorismatmutase-Prephenatdehydrogenasen bzw. deren kodierende Nukleinsäuren sind insbesondere Nukleinsäuren bakterieller Herkunft, wie beispielsweise, die Chorismatmutase-Prephenatdehydrogenasegene aus Erwinia herbicola (Accession X60420; Dieses Protein kann durch Deletion eines 109 Bp Bereiches am 5'Ende auch in eine monofunktionelle Prephenatdehydrogenase umgewandelt werden und dann beispielsweise wie vorstehend beschrieben als Prephenatdehydrogenase verwendet werden) oder Bordetella bronchiseptica (Accession AAF01289) oder lassen sich beispielsweise aus verschiedenen Organismen deren genomische Sequenz bekannt ist durch Homologievergleiche der Aminosäuresequenzen oder der entsprechenden rückübersetzten Nukleinsäuresequenzen aus Datenbanken mit der SEQ ID NO. 2 oder den anderen vorstehend beschriebenen Sequenzen leicht auffinden, wie beispielsweise die potentielle Chorismatmutase-Prephenatdehydrogenasegene aus Methanococcus janaschii (Accession Q58029).

Besonders bevorzugt verwendete Nukleinsäuren kodieren eine Chorismatmutase-Prephenatdehydrogenase aus Bakterien.

Eine besonders bevorzugt verwendete Nukleinsäure hat die Sequenz SEQ ID NO. 1. Diese Nukleinsäure stellt eine prokaryontische genomische DNA aus E. Coli K12 dar, die die Chorismatmutase-Prephenatdehydrogenase der Sequenz SEQ ID NO. 2, auch tyrA-Gen genannt, kodiert.

Figur 1 zeigt beispielsweise das Biosyntheseschema ausgehend von Erythrose-4-Phosphat zu den Tocopherolen. Durch die zusätzliche Expression eines Chorismatmutase-Prephenatdehydrogenase-Gens wird der Shikimatweg des Wildtyps genetisch verändert und der Metabolitfluß zu Hydroxyphenylpyruvat erhöht. Das nun vermehrt zur Verfügung stehende Hydroxyphenylpyruvat wird weiter in Richtung Tocopherole umgesetzt. Ein erhöhter Gehalt an Hydroxyphenylpyruvat führt zu einer erhöhten Umsetzung Richtung Vitamin E und/oder Vitamin K. Vorzugsweise führt ein erhöhter Gehalt an Hydroxyphenylpyruvat im erfindungsgemäßen Verfahren zu einer Erhöhung des Vitamin E-Gehalts.

Im erfindungsgemäßen Verfahren zur Herstellung von Vitamin E wird vorzugsweise dem Kultivierungsschritt der transgenen Organismen ein Ernten der Organismen und ein Isolieren der Feinchemikalien aus den Organismen angeschlossen.

Das Ernten der Organismen erfolgt in an sich bekannter Weise dem jeweiligen Organismus entsprechend. Mikroorganismen, wie Bakterien, Moose, Hefen und Pilze oder Pflanzenzellen, die durch Fermentation in flüssigen Nährmedien kultiviert werden, können beispielsweise durch Zentrifugieren, Dekantieren oder Filtrieren abgetrennt werden. Pflanzen werden in an sich bekannter Weise auf Nährböden gezogen und entsprechend geerntet.

Die Isolierung der Feinchemikalien aus der geernteten Biomasse erfolgt in an sich bekannter Weise, beispielsweise durch Extraktion und gegebenenfalls weiterer chemische oder physikalischer Reinigungsprozesse, wie beispielsweise Fällungsmethoden, Kristallographie, thermische Trennverfahren, wie Rektifizierverfahren oder physikalische Trennverfahren, wie beispielsweise Chromatographie.

Die Isolierung von Vitamin E aus Öl-haltigen Pflanzen erfolgt beispielsweise bevorzugt durch chemische Umwandlung und Destillation aus Pflanzenölen oder aus den bei der Desodorierung pflanzlicher Öle anfallenden Wasserdampfdestillaten (Dämpferkondensate).

Weitere Isolierverfahren von Vitamin E aus Dämpferkondensaten sind beispielsweise in DE 31 26 110 A1, EP 171 009 A2, GB 2 145 079, EP 333 472 A2 und WO 94/05650 beschrieben.

Die Herstellung der transgenen Organismen, insbesondere Pflanzen erfolgt vorzugsweise durch Transformation der Ausgangsorganismen, insbesondere Pflanzen, mit einem Nukleinsäurekonstrukt, das die vorstehend beschriebenen Nukleinsäuren, insbesondere die Nukleinsäuren codierend eine Chorismatmutase, eine Prephenatdehydrogenase oder eine Chorismatmutase-Prephenatdehydrogenase oder die vorstehen beschriebenen Nukleinsäurekonstrukte, insbesondere das Nukleinsäurekonstrukt, kodierend für ein plastidäres Transitpeptid und eine cytosolische Chorismatmutase enthält.

Diese Nukleinsäurekonstrukte, in denen die kodierende Nukleinsäuresequenz oder das kodierende Nukleinsäurekonstrukt mit einem oder mehreren Regulationssignalen funktionell verknüpft sind, die die Transkription und Translation in Organismen, insbesondere in Pflanzen gewährleisten, werden im folgenden auch Expressionskassetten genannt.

Dementsprechend beschreibt die Erfindung ferner als Expressionskassette fungierende Nukleinsäurekonstrukte, enthaltend eine, vorstehend beschriebene Nukleinsäure, insbesondere die Nukleinsäure codierend eine Chorismatmutase, eine Prephenatdehydrogenase oder eine Chorismatmutase-Prephenatdehydrogenase oder die vorstehend beschriebenen Nukleinsäurekonstrukte, insbesondere das Nukleinsäurekonstrukt, kodierend für ein plastidäres Transitpeptid und eine cytosolische Chorismatmutase, die mit einem oder mehreren Regulationssignalen funktionell verknüpft sind, die die Transkription und Translation im Wirtsorganismus, insbesondere in Pflanzen gewährleisten. Vorzugsweise enthält die Expressionskassette eine Nukleinsäure kodierend ein plastidäres Transitpeptid, das die Lokalisation in Plastiden gewährleistet.

Die Expressionskassetten beinhalten Regulationssignale, also regulative Nukleinsäuresequenzen, welche die Expression der kodierenden Sequenz in der Wirtszelle steuern. Gemäß einer bevorzugten Ausführungsform umfaßt eine Expressionskassette stromaufwärts, d.h. am 5'-Ende der kodierenden Sequenz, einen Promotor und stromabwärts, d.h. am 3'-Ende, ein Polyadenylierungssignal und gegebenenfalls weitere regulatorische Elemente, welche mit der dazwischenliegenden kodierenden Sequenz für mindestens eines der vorstehend beschriebenen Gene operativ verknüpft sind. Unter einer operativen Verknüpfung versteht man die sequenzielle Anordnung von Promotor, kodierender Sequenz, Terminator und ggf. weiterer regulativer Elemente derart, daß jedes der regulativen Elemente seine Funktion bei der Expression der kodierenden Sequenz bestimmungsgemäß erfüllen kann.

Im folgenden werden beispielhaft die bevorzugten Nukleinsäurekonstrukte, Expressionskassetten für Pflanzen und Verfahren zur Herstellung von transgenen Pflanzen beschrieben.

Die zur operativen Verknüpfung bevorzugten aber nicht darauf beschränkten Sequenzen sind Targeting-Sequenzen zur Gewährleistung der subzellulären Lokalisation im Apoplasten, in der Vakuole, in Plastiden, im Mitochondrium, im Endoplasmatischen Retikulum (ER), im Zellkern, in Ölkörperchen oder anderen Kompartimenten und Translationsverstärker wie die 5'-Führungssequenz aus dem Tabak-Mosaik-Virus (Gallie et al., Nucl. Acids Res. 15 (1987), 8693 -8711).

Als Promotor der Expressionskassette ist grundsätzlich jeder Promotor geeignet, der die Expression von Fremdgenen in Pflanzen steuern kann. Vorzugsweise verwendet man insbesondere einen pflanzlichen Promotor oder einen Promotor, der einem Pflanzenvirus entstammt. Insbesondere bevorzugt ist der CaMV 35S-Promotor aus dem Blumenkohl-Mosaik-Virus (Franck et al., Cell 21 (1980), 285-294). Dieser Promotor enthält bekanntlich unterschiedliche Erkennungssequenzen für transkriptionale Effektoren, die in ihrer Gesamtheit zu einer permanenten und konstitutiven Expression des eingeführten Gens führen (Benfey et al., EMBO J. 8 (1989), 2195-2202).

Die Expressionskassette kann auch einen chemisch induzierbaren Promotor enthalten, durch den die Expression des exogenen tyrA-Gens in der Pflanze zu einem bestimmten Zeitpunkt gesteuert werden kann. Derartige Promotoren wie z.B. der PRP1-Promotor (Ward et al., Plant. Mol. Biol. 22 (1993), 361-366), ein durch Salizylsäure induzierbarer Promotor (WO 95/19443), ein durch Benzenesulfonamid-induzierbarer (EP-A 388186), ein durch Tetrazyklin-induzierbarer (Gatz et al., (1992) Plant J. 2, 397-404), ein durch Abscisinsäure-induzierbarer (EP-A 335528) bzw. ein durch Ethanol- oder Cyclohexanon-induzierbarer (WO 93/21334) Promotor können u.a. verwendet werden.

Weiterhin sind insbesondere solche Promotoren bevorzugt, die die Expression in Geweben oder Pflanzenteilen sicherstellen, in denen beispielsweise die Biosynthese der entsprechenden Feinchemikalien, insbesondere Vitamin E bzw. dessen Vorstufen stattfindet. Insbesondere zu nennen sind Promotoren, die eine blattspezifische Expression gewährleisten. Zu nennen sind der Promotor der cytosolischen FBPase aus Kartoffel oder der ST-LSI Promotor aus Kartoffel (Stockhaus et al., EMBO J. 8 (1989), 2445-245).

Mit Hilfe eines samenspezifischen Promotors konnte ein Fremdprotein stabil bis zu einem Anteil von 0,67 % des gesamten löslichen Samenproteins in den Samen transgener Tabakpflanzen exprimiert werden (Fiedler und Conrad, Bio/Technology 10 (1995), 1090-1094). Die Expressionskassette kann daher beispielsweise einen samenspezifischen Promotor (bevorzugt den Phaseolin-Promotor (US 5504200), den USP- (Baumlein, H. et al., Mol. Gen. Genet. (1991) 225 (3), 459-467), LEB4-Promotor (Fiedler und Conrad, 1995), Sucrose-Bindeprotein-Promotor, das LEB4-Signalpeptid, das zu exprimierende Gen und ein ER-Retentionssignal enthalten.

Die Herstellung einer Expressionskassette erfolgt vorzugsweise durch Fusion eines geeigneten Promotors mit einer geeigneten, vorstehend beschriebenen Nukleinsäure-Sequenz, insbesondere der Nukleinsäure-Sequenz codierend eine Chorismatmutase, eine Prephenatdehydrogenase oder eine Chorismatmutase-Prephenatdehydrogenase und vorzugsweise einer zwischen Promotor und Nukleinsäure-Sequenz inserierten Nukleinsäure, die für ein chloroplastenspezifisches Transitpeptid kodiert, sowie einem Polyadenylierungssignal nach gängigen Rekombinations- und Klonierungstechniken, wie sie beispielsweise in T. Maniatis, E. F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley-Interscience (1987) beschrieben sind.

Insbesondere bevorzugt sind insertierte Sequenzen, die, wie vorstehend für die Chorismatmutase beschrieben, ein Targeting in den Plastiden gewährleisten.

Es können auch Expressionskassetten verwendet werden, deren Nukleinsäure-Sequenz für ein Fusionsprotein, insbesondere ein Chorismatmutase-, Prephenatdehydrogenase- oder Chorismatmutase-Prephenatdehydrogenase-Fusionsprotein kodiert, wobei ein Teil des Fusionsproteins ein Transitpeptid ist, das die Translokation des Polypeptides steuert. Bevorzugt sind für die Chloroplasten spezifische Transitpeptide, welche nach Translokation der Proteine, insbesondere der Chorismatmutase, Prephenatdehydrogenase oder Chorismatmutase-Prephenatdehydrogenase in die Chloroplasten vom Proteinteil, insbesondere vom Chorismatmutase-, Prephenatdehydrogenase- bzw. Chorismatmutase-Prephenatdehydrogenase-Teil enzymatisch abgespalten werden. Insbesondere bevorzugt ist das Transitpeptid, das von der plastidären *Nicotiana tabacum* Transketolase oder einem anderen Transitpeptid (z.B. dem Transitpeptid der kleinen Untereinheit der Rubisco oder der Ferredoxin NADP Oxidoreduktase als auch der Isopentenylpyrophosphat Isomerase-2) oder dessen funktionellem Äquivalent abgeleitet ist.

Für die Verwendung der cytosolischen Chorismatmutase bzw. der Nukleinsäure, codierend eine cytosolische Chorismatmutase ist, wie vorstehend beschrieben, insbesondere die Verwendung des Transitpeptids der plastidären Chorismatmutase, bzw. deren kodierende Nukleinsäure bevorzugt.

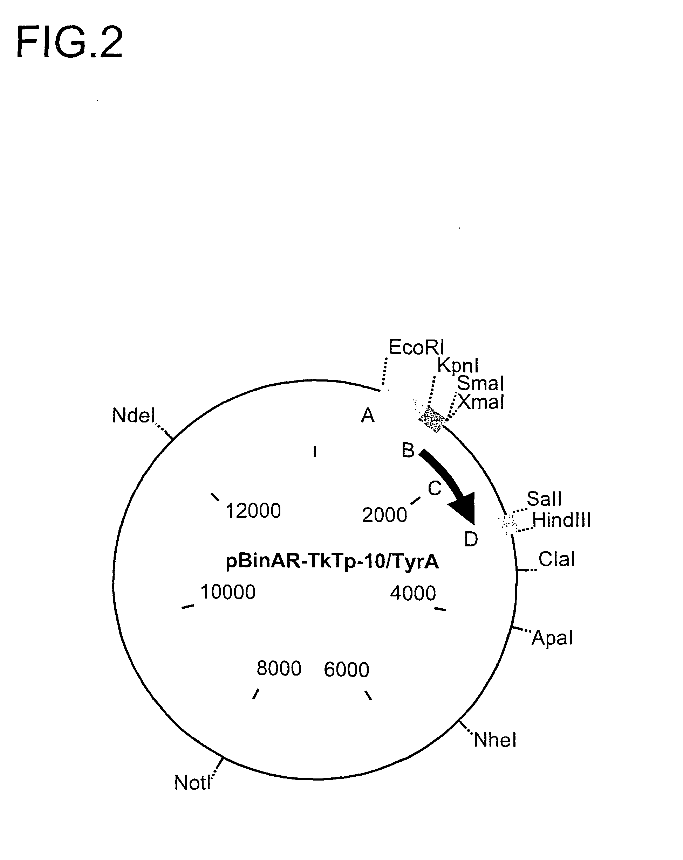

Besonders bevorzugt bei der erfindungsgemäßen Verwendung der anderen, erfindungsgemäßen Nukleinsäuren sind DNA-Sequenzen von drei Kassetten des Plastiden-Transitpeptids der plastidären Transketolase aus Tabak in drei Leserastern als KpnI/BamHI Fragmente mit einem ATG-Codon in der NcoI Schnittstelle:
pTP09
pTP10
pTP11
   Ein weiteres Beispiel für ein plastidäres Transitpeptid ist das Transitpeptid der plastidären Isopentenyl-pyrophosphat Isomerase-2 (IPP-2) aus Arabisopsis thaliana.
   Die Nukleinsäuren, insbesondere die Nukleinsäuren codierend eine Chorismatmutase, eine Prephenatdehydrogenase oder eine Chorismatmutase-Prephenatdehydrogenase können synthetisch hergestellt oder natürlich gewonnen sein oder eine Mischung aus synthetischen und natürlichen Nukleinsäure-Bestandteilen enthalten, sowie aus verschiedenen heterologen Genabschnitten verschiedener Organismen bestehen.
   Bevorzugt sind, wie vorstehend beschrieben, synthetische Nukleotid-Sequenzen mit Kodons, die von Pflanzen bevorzugt werden. Diese von Pflanzen bevorzugten Kodons können aus Kodons mit der höchsten Proteinhäufigkeit bestimmt werden, die in den meisten interessanten Pflanzenspezies exprimiert werden.
   Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente miteinander können an die Fragmente Adaptoren oder Linker angesetzt werden.
   Zweckmäßigerweise können die Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zur Wirtspflanze sein. Die Expressionskassette beinhaltet vorzugsweise in der 5'-3'-Transkriptionsrichtung den Promotor, eine kodierende Nukleinsäuresequenz oder ein Nukleinsäurekonstrukt und eine Region für die transkriptionale Termination. Verschiedene Terminationsbereiche sind gegeneinander beliebig austauschbar.
   Ferner können Manipulationen, die passende Restriktionsschnittstellen bereitstellen oder die überflüssige DNA oder Restriktionsschnittstellen entfernen, eingesetzt werden. Wo Insertionen, Deletionen oder Substitutionen wie z.B.
   Transitionen und Transversionen in Frage kommen, können in vitro-Mutagenese, "primerrepair", Restriktion oder Ligation verwendet werden.
   Bei geeigneten Manipulationen, wie z.B. Restriktion, "chewing-back" oder Auffüllen von Überhängen für "bluntends", können komplementäre Enden der Fragmente für die Ligation zur Verfügung gestellt werden.
   Bevorzugte Polyadenylierungssignale sind pflanzliche Polyadenylierungssignale, vorzugsweise solche, die im wesentlichen T-DNA-Polyadenylierungssignale aus Agrobacterium tumefaciens, insbesondere des Gens 3 der T-DNA (Octopin Synthase) des Ti-Plasmids pTiACH5 entsprechen (Gielen et al., EMBO J. 3 (1984), 835 ff) oder funktionelle Äquivalente.
   Ferner betrifft die Erfindung die Verwendung der vorstehend beschriebenen Nukleinsäuren, insbesondere der Nukleinsäuren kodierend eine Chorismatmutase, eine Prephenatdehydrogenase oder eine Chorismatmutase-Prephenatdehydrogenase oder der vorstehend beschriebenen Nukleinsäurekonstrukte oder Proteine, insbesondere der Chorismatmutasen, der Prephenatdehydrogenasen oder der Chorismatmutase-Prephenatdehydrogenasen zur Herstellung von transgenen Pflanzen.
   Vorzugsweise weisen diese transgenen Pflanze gegenüber dem Wildtyp einen erhöhten Gehalt an Feinchemikalien, insbesondere an Ubichinon, Vitamin E und/oder Vitamin K, vorzugsweise an Vitamin E auf.
   Es ist bekannt, daß Pflanzen mit einem hohen Vitamin-E-Gehalt eine erhöhte Resistenz gegenüber abiotischem Streß aufweisen. Unter abiotischem Streß wird beispielsweise Kälte, Frost, Trockenheit, Hitze und Salz verstanden.
   Daher betrifft die Erfindung weiterhin die Verwendung der vorstehend erwähnten Nukleinsäuren zur Herstellung transgener Pflanzen, die gegenüber dem Wildtyp eine erhöhte Resistenz gegenüber abiotischem Streß aufweisen.
   Die vorstehend beschriebenen Proteine und Nukleinsäuren können zur Herstellung von Feinchemikalien in transgenen Organismen, vorzugsweise zur Herstellung von Vitamin E, Vitamin K und/oder Ubichinon, insbesondere Vitamin E in transgenen Pflanzen verwendet werden.
   Die Übertragung von Fremdgenen in das Genom eines Organismus, insbesondere einer Pflanze wird als Transformation bezeichnet. Dazu können insbesondere bei Pflanzen an sich bekannte Methoden zur Transformation und Regeneration von Pflanzen aus Pflanzengeweben oder Pflanzenzellen zur transienten oder stabilen Transformation genutzt werden.
   Geeignete Methoden zur Transformation von Pflanzen sind die Protoplastentransformation durch Polyethylenglykol-induzierte DNA-Aufnahme, das biolistische Verfahren mit der Genkanone - die sogenannte particle bombardment Methode, die Elektroporation, die Inkubation trockener Embryonen in DNA-haltiger Lösung, die Mikroinjektion und der, vorstehend beschriebene, durch Agrobacterium vermittelte Gentransfer. Die genannten Verfahren sind beispielsweise in B. Jenes et al., Techniques for Gene Transfer, in: Transgenic Plants, Vol. 1, Engineering and Utilization, herausgegeben von S.D. Kung und R. Wu, Academic Press (1993), 128-143 sowie in Potrykus, Annu. Rev. Plant Physiol. Plant Molec. Biol. 42 (1991), 205-225) beschrieben.
   Vorzugsweise wird das zu exprimierende Konstrukt in einen Vektor kloniert, der geeignet ist, Agrobacterium tumefaciens zu transformieren, beispielsweise pBin19 (Bevan et al., Nucl. Acids Res. 12 (1984), 8711).
   Dementsprechend beschreibt die Erfindung weiterhin Vektoren enthaltend die vorstehend beschriebenen Nukleinsäuren, Nukleinsäurekonstrukte oder Expressionskassetten.
   Mit einer Expressionskassette transformierte Agrobakterien können in bekannter Weise zur Transformation von Pflanzen verwendet werden, z.B. indem verwundete Blätter oder Blattstücke in einer Agrobakterienlösung gebadet und anschließend in geeigneten Medien kultiviert werden.
   Die Expressionskassette kann über die Pflanzen hinaus auch zur Transformation von Bakterien, insbesondere Cyanobakterien, Moosen, Hefen, filamentösen Pilzen und Algen eingesetzt werden.
   Zur bevorzugten Herstellung von genetisch veränderten Pflanzen, im folgenden auch transgene Pflanzen bezeichnet, wird die fusionierte Expressionskassette, die ein Protein, insbesondere eine Chorismatmutase, Prephenatdehydrogenase oder eine Chorismatmutase-Prephenatdehydrogenase kodiert, vorzugsweise in einen Vektor, beispielsweise pBin19, kloniert, der geeignet ist, *Agrobacterium tumefaciens* zu transformieren.
   Mit einem solchen Vektor transformierte Agrobakterien können dann in bekannter Weise zur Transformation von Pflanzen, insbesondere von Kulturpflanzen verwendet werden, indem beispielsweise verwundete Blätter oder Blattstücke in einer Agrobakterienlösung gebadet und anschließend in geeigneten Medien kultiviert werden.
   Die Transformation von Pflanzen durch Agrobakterien ist unter anderem bekannt aus F.F. White, Vectors for Gene Transfer in Higher Plants; in Transgenic Plants, Vol. 1, Engineering and Utilization, herausgegeben von S.D. Kung und R. Wu, Academic Press, 1993, S. 15-38. Aus den transformierten Zellen der verwundeten Blätter bzw. Blattstücke können in bekannter Weise transgene Pflanzen regeneriert werden, die ein in die Expressionskassette integriertes Gen für die Expression eines Gens, insbesondere einer Nukleinsäure kodierend eine eine Chorismatmutase, eine Prephenatdehydrogenase oder eine Chorismatmutase-Prephenatdehydrogenase enthalten.
   Zur Transformation einer Wirtspflanze mit einer für eine Chorismatmutase, Prephenatdehydrogenase oder Chorismatmutase-Prephenatdehydrogenase kodierenden Nukleinsäure wird eine Expressionskassette als Insertion in einen rekombinanten Vektor eingebaut, dessen Vektor-DNA zusätzliche funktionelle Regulationssignale, beispielsweise Sequenzen für Replikation oder. Integration enthält. Geeignete Vektoren sind unter anderem in "Methods in Plant Molecular Biology and Biotechnology" (CRC Press), Kap. 6/7, S. 71-119 (1993) beschrieben.
   Beispielhaft kann die pflanzliche Expressionskassette in ein Derivat des Transformationsvektors pBin-19 mit 35s Promotor (Bevan, M., Nucleic Acids Research 12: 8711-8721 (1984)) eingebaut werden. Figur 2 zeigt ein Derivat des Transformationsvektors pBin-19 mit samenspezifischem Legumin B4-Promotor.
   Unter Verwendung der oben zitierten Rekombinations- und Klonierungstechniken können die Expressionskassetten in geeignete Vektoren kloniert werden, die ihre Vermehrung, beispielsweise in E. coli, ermöglichen. Geeignete Klonierungsvektoren sind u.a. pBR332, pUC-Serien, M13mp-Serien und pACYC184. Besonders geeignet sind binäre Vektoren, die sowohl in *E. coli* als auch in Agrobakterien replizieren können.
   Die Erfindung betrifft daher die Verwendung der vorstehend beschriebenen Nukleinsäuren, insbesondere die Nukleinsäuren koodierend eine Chorismatmutase, Prephenatdehydrogenase oder eine Chorismatmutase-Prephenatdehydrogenase, der vorstehend beschriebenen Nukleinsäurekonstrukte, insbesondere der Expressionskassetten zur Herstellung von genetisch veränderten Pflanzen oder zur Transformation von Pflanzen, -zellen, -geweben oder Pflanzenteilen. Vorzugsweise ist Ziel der Verwendung die Erhöhung des Gehaltes der Pflanze oder Pflanzenteile an Vitamin E.
   Dabei kann je nach Wahl des Promotors die Expression spezifisch in den Blättern, in den Samen, Blütenblättern oder anderen Teilen der Pflanze erfolgen.
   Dementsprechend betrifft die Erfindung ferner ein Verfahren zur Herstellung von genetisch veränderten Organismen indem man eine vorstehend beschriebene Nukleinsäure oder ein vorstehend beschriebenes Nukleinsäurekonstrukt in das Genom des Ausgangsorganismus einführt.
   Vorzugsweise betrifft die Erfindung ein Verfahren zur Transformation einer Pflanze dadurch gekennzeichnet, daß man Expressionskassetten enthaltend Nukleinsäuresequenzen, kodierend eine kodierend eine Chorismatmutase, Prephenatdehydrogenase oder eine Chorismatmutase-Prephenatdehydrogenase in eine Pflanzenzelle oder Protoplasten von Pflanzen einbringt und diese zu ganzen Pflanzen regeneriert.
   Die Erfindung beschreibt auch die genetisch veränderte Organismen, wobei die genetische Veränderung den Metabolitfluß des Shikimatweges gegenüber dem Wildtyp verändert und der Organismus gegenüber dem Wildtyp einen veränderten Gehalt an Feinchemikalien aufweist.
   Wie vorstehend erwähnt, weisen bevorzugte genetisch veränderte. Organismen einen erhöhten Gehalt an Feinchemikalien, insbesondere einen erhöhten Gehalt an Vitamin E, Vitamin K und Ubichinon, vorzugsweise einen erhöhten Gehalt an Vitamin E gegenüber dem Wildtyp auf.
   Unter einem genetisch veränderter Organismus wird insbesondere ein Organismus verstanden, bei dem die genetische Veränderung die Genexpression einer Nukleinsäure kodierend eine Chorismatmutase, Prephenatdehydrogenase oder Choroismatmutase-Prephenatdehydrogenase gegenüber einem Wildtyp
   für den Fall, daß der Ausgangsorganismus die entsprechende Nukleinsäure enthält, erhöht oder
   für den Fall, daß der Ausgangsorganismus die entsprechende Nukleinsäure nicht enthält, verursacht.

Als Organismen und zur Herstellung von Organismen mit einem erhöhten Gehalt an Feinchemikalien im Vergleich zum Wildtyp werden in einer bevorzugten Ausführungsform der erfindungsgemäßen Verfahren und Verwendungen, wie vorstehend erwähnt, photosynthetisch aktive Organismen wie beispielsweise Cyanobakterien, Moose, Algen oder Pflanzen, besonders bevorzugt Pflanzen als Ausgangsorganismen und dementsprechend auch als genetisch veränderte Organismen verwendet.

Pflanzen im Sinne der Erfindung sind insbesondere mono- und dikotyle Pflanzen.

Bevorzugte Pflanzen sind Tagetes, Sonnenblume, Arabidopsis, Tabak, Roter Pfeffer, Soja, Tomate, Aubergine, Paprika, Möhre, Karotte, Kartoffel, Mais, Salate und Kohlarten, Getreide, Alfalfa, Hafer, Gerste, Roggen, Weizen, Triticale, Hirse, Reis, Luzerne, Flachs, Baumwolle, Hanf, Brassicacaen wie beispielsweise Raps oder Canola, Zuckerrübe, Zuckerrohr, Nuß- und Weinspezies oder Holzgewächse wie beispielsweise Espe oder Eibe.

Besonders bevorzugt sind *Arabidopsis thaliana*, *Tagetes erecta*, *Brassica napus, Nicotiana tabacum*, Canola, Kartoffeln sowie weitere Ölsaaten, wie beispielsweise Soja.

Die genetisch veränderten Organismen, insbesondere Pflanzen können, wie vorstehend beschrieben zur Herstellung von Feinchemikalien, insbesondere zur Herstellung von Vitamin E verwendet werden.

Von Menschen und Tieren verzehrbare, genetisch veränderte Pflanzen mit erhöhtem Gehalt an Feinchemikalien, insbesondere mit einem erhöhten Gehalt an Vitamin-E, können auch beispielsweise direkt oder nach an sich bekannter Prozessierung als Nahrungsmittel oder Futtermittel verwendet werden.

Erhöhung des Gehaltes an Feinchemikalien bedeutet im Rahmen der vorliegenden Erfindung die künstlich erworbene Fähigkeit einer erhöhten Biosyntheseleistung dieser Verbindungen in der Pflanze gegenüber der nicht gentechnisch modifizierten Pflanze für die Dauer mindestens einer Pflanzengeneration.

Unter einem erhöhten Gehalt an Vitamin E wird in der Regel ein erhöhter Gehalt an Gesamt-Tocopherol verstanden. Unter einem erhöhten Gehalt an Vitamin E wird aber auch insbesondere ein veränderter Gehalt der vorstehend beschriebenen 8 Verbindungen mit Tocopherolaktivität verstanden.

Beispielsweise führt das Einbringen eines Chorismatmutase-Prephenatdehydrogenase-Gens in Pflanzen überraschenderweise zu einem besonders erhöhten Anstieg des Gehalts an Tocotrienolen.

Im Falle der Erhöhung des Gehalts an Vitamin E kann sowohl der Gehalt an Tocopherolen oder Tocotrienolen gesteigert werden. Vorzugsweise wird der Gehalt an Tocopherolen gesteigert. Aber es ist auch möglich unter bestimmten Bedingungen vorzugsweise den Gehalt an Tocotrienolen zu steigern.

Der Biosyntheseort von Vitamin E beispielsweise ist in Pflanzen unter anderem das Blattgewebe, so daß eine blattspezifische Expression der erfindungsgemäßen Nukleinsäuren, insbesondere der Nukleinsäuren codierend eine Chorismatmutase, eine Prephenatdehydrogenase oder eine Chorismatmutase-Prephenatdehydrogenase sinnvoll ist. Dies ist jedoch nicht einschränkend, da die Expression auch in allen übrigen Teilen der Pflanze - besonders in fetthaltigen Samen - gewebespezifisch erfolgen kann.

Eine weitere bevorzugte Ausführungsform der erfindungsgemäßen Verfahren und Verwendungen betrifft deshalb eine samenspezifische Expression der erfindungsgemäßen Nukleinsäuren, insbesondere der Nukleinsäuren codierend eine Chorismatmutase, eine Prephenatdehydrogenase oder eine Chorismatmutase-Prephenatdehydrogenases.

Darüber hinaus ist eine konstitutive Expression von exogenen Chorismatmutase-, Prephenatdehydrogenase- oder Chorismatmutase-Prephenatdehydrogenase-Genen von Vorteil. Andererseits kann aber auch eine induzierbare Expression wünschenswert erscheinen.

Die Wirksamkeit der Expression des transgen exprimierten Chorismatmutase-, Prephenatdehydrogenase- oder Chorismatmutase-Prephenatdehydrogenase-Gens kann beispielsweise *in vitro* durch Sproßmeristemvermehrung ermittelt werden. Zudem kann eine in Art und Höhe veränderte Expression des Chorismatmutase-, Prephenatdehydrogenase- oder Chorismatmutase-Prephenatdehydrogenase-Gens und deren Auswirkung auf die Vitamin E-Biosyntheseleistung an Testpflanzen in Gewächshausversuchen getestet werden.

Die Erfindung wird durch die nun folgenden Beispiele erläutert, ist aber nicht auf diese beschränkt:
Allgemeine Experimentelle Bedingungen:
Sequenzanalyse rekombinanter DNA

Die Sequenzierung rekombinanter DNA-Moleküle erfolgte mit einem Laserfluoreszenz-DNA-Sequenzierer der Firma Licor (Vertrieb durch MWG Biotech, Ebersbach) nach der Methode von Sanger (Sanger et al., Proc. Natl. Acad. Sci. USA 74 (1977), 5463-5467).

### Beispiel 1 - Klonierung des tyrA-Gens kodierend für die Chorismatmutase-Prephenatdehydrogenase aus E. coli K12

Die DNA kodierend für das tyrA-Gen wurde mittels *polymerase chain reaction* (PCR) aus *E. coli* K12 unter Verwendung eines sense spezifischen Primers (tyrA5' SEQ ID NO. 10) und eines antisense spezifischen Primers (tyrA3' SEQ ID NO. 9) amplifiziert.

Die PCR Bedingungen waren die folgenden:

Die PCR erfolgte in einem 50µl Reaktionsansatz in dem enthalten war:
- 2 µl einer *E. coli* K12 Zellsuspension
- 0,2 mM dATP, dTTP, dGTP, dCTP
- 1,5 mM Mg(OAc)₂
- 5 µg Rinderserum-Albumin
- 40 pmol tyrA5'
- 40 pmol tyrA3'
- 15 µl 3,3 x rTth DNA Polymerase XLPuffer (PE Applied Biosystems)
- 5 U rTth DNA Polymerase XL (PE Applied Biosystems)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 55°C (Annealing)
Schritt 9: 2 Minuten 72°C (Elongation)
30 Wiederholungen der Schritte 2 bis 4
Schritt 5: 10 Minuten 72°C (Post-Elongation)
Schritt 6: 4°C (Warteschleife)

Das Amplikon wurde unter Verwendung von Standardmethoden in den PCR Klonierungsvektor pGEM-T (Promega) kloniert. Die Identität des erzeugten Amplikons wurde durch Sequenzierung unter Verwendung des M13F (-40) Primers bestätigt.

### Beispiel 2 - Herstellung von Expressionskassetten enthaltend das tyrA-Gen, kodierend für die Chorismatmutase-Prephenatdehydrogenase aus E.coli K12

Transgene *Nicotiana tabacum* und *Arabidopsis thaliana* Pflanzen wurden erzeugt, die die Chorismatmutase-Prephenatdehydrogenase aus *E.coli* K12 unter Kontrolle des konstitutiven 35S-Promotor des CaMV (Blumenkohlmosaikvirus) (Franck et al., Cell 21: 285-294, 1980) exprimieren. Die Grundlage des zur konstitutiven Expression der Chorismatmutase-Prephenatdehydrogenase aus *E.coli* K12 erzeugten Plasmides war der pBinAR-TkTp-10 (Ralf Badur, Dissertation Universität Göttingen, 1998). Dieser Vektor ist ein Derivat des pBinAR (Höfgen und Willmitzer, Plant Sci. 66: 221-230, 1990) und enthält den 35S-Promotor des CaMV (Blumenkohlmosaikvirus) (Franck et al., 1980) das Terminations-signal des Octopin-Synthase Gens (Gielen et al., EMBO J. 3: 835-846, 1984) sowie die für das Transitpeptid der *Nicotiana tabacum* plastidären Transketolase kodierenden DNA Sequenz. Die unter Berücksichtigung des korrekten Leserasters erfolgte Klonierung der Chorismatmutase-Prephenatdehydrogenase aus *E.coli* K12 in diesen Vektor, erzeugt eine Translationsfusion der Chorismatmutase-Prephenatdehydrogenase mit dem plastidären Transitpeptid. Dadurch erfolgt ein Transport des Transgens in die Plastiden.

Zur Erstellung dieses Plasmides wurde das tyrA-Gen unter Verwendung der flankierenden SmaI bzw. SalI Restriktionsschnittstellen aus dem Plasmid pGEM-T/tyrA isoliert. Dieses Fragment wurde unter Anwendung von Standardmethoden in einen SmaI / SalI geschnittenen pBinAR-TkTp-10 ligiert (siehe Figur 2). Dieses Plasmid (pBinAR-TkTp-10/tyrA) wurde zur Erzeugung transgener *Nicotiana tabacum* und *A.thaliana* Pflanzen verwendet.

Fragment A (529 bp) in Figur 2 beinhaltet den 35S-Promotor des CaMV (Nukleotide 6909 bis 7437 des Blumenkohlmosaikvirus), Fragment B (245bp) kodiert für das Transitpeptid der *Nicotiana tabacum* Transketolase, Fragment C (1232 Bp) kodiert für das tyrA-Gen aus *E. coli* K12, Fragment D (219Bp) kodiert für das Terminationssignal des Octopin-Synthase Gens.

### Beispiel 3 - Erzeugung von Nukleinsäurekonstrukten zur Expression der Chorismatmutase-Prephenatdehydrogenase aus E.coli K12 unter Kontrolle eines samenspezifischen Promotors

Zur Herstellung von chimären DNA Konstrukten zur Erzeugung transgener *Arabidopsos thaliana, Nicotiana tabacum bzw. Brassica napus* Pflanzen, die die Chorismatmutase-Prephenatdehydrogenase aus *E.coli* K12 unter Kontrolle eines samenspezifischen Promotors exprimieren, wurde der Vektor pPTVkanLeP-IPP-TP-9 verwendet.

Dieser Vektor ist ein Derivat des pGPTVkan (D. Becker, E. Kemper, J. Schell, R. Masterson. Plant Molecular Biology 20: 1195-1197, 1992) dem das *uidA* Gen deletiert wurde. Stattdessen enthält der Vektor pPTVkanLeP-IPP-TP-9 den samenspezifischen Promotor des Legumin B4 Gens (Kafatos et al., Nuc. Acid. Res., 14 (6) : 2707-2720, 1986), die Sequenz kodierend für das Transitpeptid der *A.thaliana* plastiden-spezifischen Isopentenyl-pyrophosphat Isomerase-2 (IPP-2) (Badur, unveröffentlicht) und das Terminationssignal der Nopalinsynthase aus *A. tumefaciens* (Depicker et al., J. Mol. Appl. Genet. 1, 561-73, 1982).

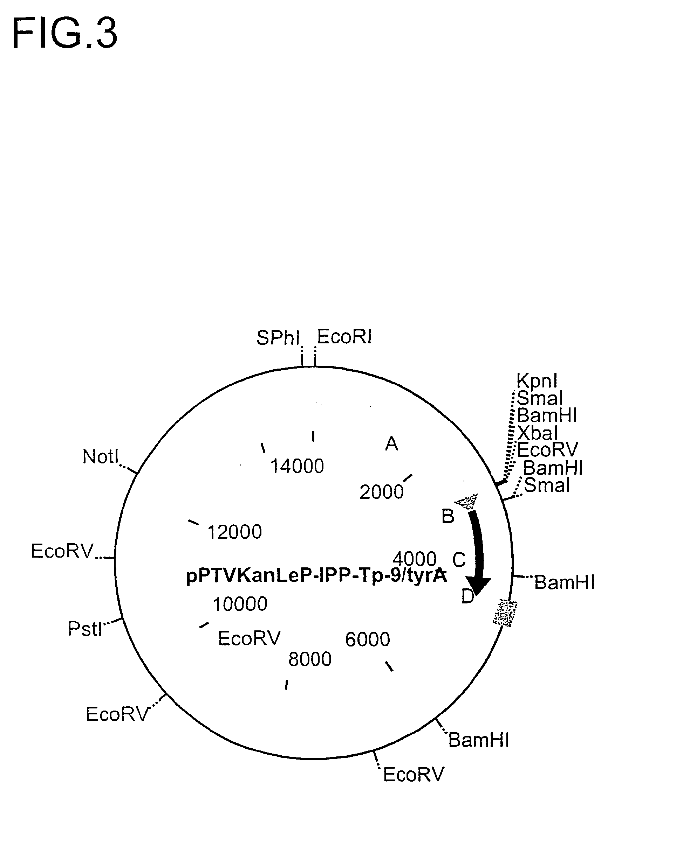

Das Nukleinsäure-Fragment kodierend für die *tyrA* aus *E. coli* K12 wurde als SmaI/SalI Fragment mit durch die T4-Polymerase aufgefüllten stumpfen Enden in den Vektor pPTVkanLeP-IPP-TP-9 (Figur 3) kloniert, wodurch eine Translationsfusion mit dem Transitpeptid der IPP-2 erzeugt wurde. Somit konnte ein Import der Chorismatmutase-Prephenatdehydrogenase in die Plastiden gewährleistet werden. Dieses Plasmid (pPTVkanLeP-IPP-TP-9/TyrA) wurde zur Erzeugung transgener *Nicotiana tabacum, A. thaliana* bzw. *Brassica napus* Pflanzen verwendet.

Fragment A (2700 bp) in Figur 3 beinhaltet den Promotor des Legumin B4 Gens aus *Vicia faba,* Fragment B (206bp) Fragment kodierend für das Transitpeptid der *A. thaliana* Isopentenyl-pyrophosphat-Isomerase-2. Fragment C (1234Bp) kodiert für das tyrA-Gen aus *E. coli* K12. Fragment D (272Bp) für das Terminationssignal des Nopalin-Synthase Gens. Beispiel 4 - Herstellung transgener *Arabidopis thaliana* Pflanzen, die das tyrA-Gen exprimieren

Wildtyp *Arabidopsis thaliana* Pflanzen (Columbia) wurden mit dem *Agrabacterium tumefaciens* Stamm (GV3101 [pMP90]) auf Grundlage einer modifizierten Vakuuminfiltrationsmethode transformiert (Steve Clough und Andrew Bent. Floral dip: a simplified method for Agrobacterium mediated transformation of A. thaliana. Plant J 16(6):735-43, 1998; der Bechtold, N. Ellis, J. und Pelltier, G., in: Planta Agrobacterium-mediated gene transfer by infiltration of adult Arabidopsis thaliana plants. CRAcad Sci Paris, 1993, 1144(2):204-212). Die verwendeten *Agrobacterium tumefaciens* Zellen waren im Vorfeld mit den Plasmiden pBinAR-TkTp-10/tyrA bzw. pPTVkanLeP-IPP-TP-9/tyrA (Figur 2 bzw. 3) transformiert worden.

Samen der Primärtransformanden wurden auf Grundlage der Antibiotikaresistenz selektioniert. Antibiotika resistente Keimlinge wurden in Erde gepflanzt und als vollentwickelte Pflanzen zur biochemischen Analyse verwendet.

### Beispiel 5 - Herstellung transgener Brassica napus Pflanzen, die das tyrA-Gen exprimieren

Die Herstellung transgener Raps Pflanzen orientierte sich an einem Protokoll von Bade, J.B. und Damm,B. (in Gene Transfer to Plants, Potrykus, I. und Spangenberg, G., eds, Springer Lab Manual, Springer Verlag, 1995, 30-38), in welchem auch die Zusammensetzung der verwendeten Medien und Puffer angegeben ist.

Die Transformationen erfolgten mit dem *Agrobacterium tumefaciens* Stamm GV3101 [pMP90]. Zur Transformation wurde das Plasmid pPTVkanLeP-IPP-TP-9/tyrA verwendet (Figur 3). Samen von Brassica napus var. Westar wurden mit 70 % Ethanol (v/v) oberflächensteril gemacht, 10 Minuten bei 55°C in Wasser gewaschen, in 1%iger Hypochlorit-Lösung (25 % v/v Teepol, 0,1 % v/v Tween 20) für 20 Minuten inkubiert und sechsmal mit sterilem Wasser für jeweils 20 Minuten gewaschen. Die Samen wurden drei Tage auf Filterpapier getrocknet und 10-15 Samen in einem Glaskolben mit 15 ml Keimungsmedium zur Keimung gebracht. Von mehreren Keimlingen (ca. 10 cm groß) wurden die Wurzeln und Apices entfernt und die verbleibenden Hypokotyle in ca. 6 mm lange Stücke geschnitten. Die so gewonnenen ca. 600 Explantate wurden 30 Minuten mit 50 ml Basalmedium gewaschen und in einem 300 ml Kolben überführt. Nach Zugabe von 100 ml Kallusinduktionsmedium wurden die Kulturen für 24 Stunden bei 100 U/min inkubiert.

Vom Agrobacterium Stamm wurde eine Übernachtkultur bei 29°C in Luria Broth-Medium mit Kanamycin (20 mg/1) angesetzt, davon 2 ml in 50 ml Luria Broth-Medium ohne Kanamycin für 4 Stunden bei 29°C bis zu einer OD₆₀₀ von 0,4 bis 0, 5 inkubiert. Nach der Pelletierung der Kultur bei 2000 U/min für 25 min wurde das Zellpellet in 25 ml Basalmedium resuspendiert. Die Konzentration der Bakterien in der Lösung wurde durch Zugabe von weiterem Basalmedium auf eine OD₆₀₀ von 0,3 eingestellt.

Aus den Raps-Explanten wurde das Kallus-Induktionsmedium mit sterilen Pipetten entfernt, 50 ml Agrobakterium-Lösung hinzugefügt, vorsichtig gemischt und für 20 min inkubiert. Die Agrobacterien-Suspension wurde entfernt, die Raps-Explante für 1 min mit 50 ml Kallus-Induktionsmedium gewaschen und anschließend 100 ml Kallus-Induktionsmedium hinzugefügt. Die Co-Kultivierung wurde für 24 h auf einem Rotationsschüttler bei 100 U/min durchgeführt. Die Co-Kultivierung wurde durch Wegnahme des Kallus-Induktionsmediums gestoppt und die Explante zweimal für jeweils 1 min mit 25 ml und zweimal für 60 min mit jeweils 100 ml Waschmedium bei 100 U/min gewaschen. Das Waschmedium mit den Explanten wurde in 15 cm Petrischalen überführt und das Medium mit sterilen Pipetten entfernt.

Zur Regeneration wurden jeweils 20 bis 30 Explante in 90 mm Petrischalen überführt, welche 25 ml Sproß-Induktionsmedium mit Kanamycin enthielten. Die Petrischalen wurden mit 2 Lagen Leukopor verschlossen und bei 25°C und 2000 lux bei Photoperioden von 16 Stunden Licht/8 Stunden Dunkelheit inkubiert. Alle 12 Tage wurden die sich entwickelnden Kalli auf frische Petrischalen mit Sproß-Induktionsmedium umgesetzt. Alle weiteren Schritte zur Regeneration ganzer Pflanzen wurden wie von Bade, J.B und Damm, B. (in: Gene Transfer to Plants, Potrykus, I. und Spangenberg, G., eds, Springer Lab Manual, Springer Verlag, 1995, 30-38) beschrieben durchgeführt.

### Beispiel 6 - Herstellung transgener Nicotiana tabacum Pflanzen, die das tyrA-Gen exprimieren

Zehn ml YEB-Medium mit Antibiotikum (5 g/l Rinder-Extrakt, 1 g/l Hefe-Extrakt, 5 g/l Pepton, 5 g/l Saccharose und 2 mM MgSO₄.) wurden mit einer Kolonie von *Agrobacterium tumefaciens* beimpft und über Nacht bei 28°C kultiviert. Die Zellen wurden 20 min bei 4°C, 3500 U/min in einer Tischzentrifuge pelletiert und danach in frischem YEB-Medium ohne Antibiotika unter sterilen Bedingungen resuspendiert. Die Zellsuspension wurde für die Transformation eingesetzt.

Die Wildtyp-Pflanzen aus Sterilkultur wurden durch vegetative Replikation erhalten. Dazu wurde nur die Spitze der Pflanze abgeschnitten und auf frisches 2MS-Medium in ein steriles Einweckglas überführt. Vom Rest der Pflanze wurden die Haare auf der Blattoberseite und die Mittelrippen der Blätter entfernt. Die Blätter wurden mit einer Rasierklinge in etwa 1cm² große Stücke geschnitten. Die Agrobakterienkultur wurde in eine kleine Petrischale überführt (Durchmesser 2 cm). Die Blattstücke wurden kurz durch diese Lösung gezogen und mit der Blattunterseite auf 2MS-Medium in Petrischalen (Durchmesser 9 cm) gelegt, so daß sie das Medium berührten. Nach zwei Tagen im Dunkeln bei 25°C wurden die Explantate auf Platten mit Kallusinduktionsmedium überführt und in der Klimakammer auf 28°C temperiert. Das Medium mußte alle 7 bis 10 Tage gewechselt werden. Sobald sich Kalli bildeten, wurden die Explantate in sterile Einweckgläser auf Sproßinduktionsmedium mit Claforan (0,6 % BiTec-Agar (g/v), 2,0 mg/l Zeatinribose, 0,02 mg/l Naphthylessigsäure, 0,02 mg/l Gibberelinsäure, 0,25 g/ml Claforan, 1,6 % Glukose (g/v) und 50 mg/l Kanamycin überführt. Nach etwa einem Monat trat Organogenese ein und die gebildeten Sprosse konnten abgeschnitten werden. Die Kultivierung der Sprosse wurde auf 2MS-Medium mit Claforan und Selektionsmarker durchgeführt. Sobald sich ein kräftiger Wurzelballen gebildet hatte, konnten die Pflanzen in Pikiererde getopft werden.

### Beispiel 7 - Charakterisierung der transgenen Pflanzen aus Beispiel 4, 5 und 6

Es wurden die Tocopherol- und Tocotrienol-Gehalte in Blätter und Samen der mit den beschriebenen Konstrukten transformierten Pflanzen *(Arabidopsis thaliana, Brassica napus* und *Nicotiana tabacum)* analysiert. Dazu wurden die transgenen Pflanzen im Gewächshaus kultiviert und Pflanzen die das Gen kodierend für die Chorismatmutase-Prephenatdehydrogenase aus *E.coli* K12 exprimieren auf Northern- und Western Ebene analysiert. In Blättern und Samen dieser Pflanzen wurde der Tocopherolgehalt und der Tocotrienolgehalt mittels HPLC ermittelt. In allen Fällen war der Tocopherol- und/oder Tocotrienol-Gehalt in transgenen Pflanzen, die zusätzlich ein tyrA-Gen exprimieren, im Vergleich zu nicht transformierten Pflanzen erhöht.

Tabelle 1A (junge Blätter) und 1B (seneszierende Blätter) zeigen die Gehalte [µg/gFG] an α-Tocopherol, γ-Tocopherol, α-Tocotrienol und Gesamt-Vitamin E in Blättern unterschiedlichen Alters in *Nicotiana tabacum,* cv. SNN-Wildtyp (angegebene Werte MW +/-SD, n = 9) und Pflanzen, die das Tyr A Gen aus E. coli überexprimieren.

**Tabelle A: junge Blätter**

| | α-Tocopherol | | γ-Tocopherol | | α-Tocotrienol | Gesamt Vitamin E | |
|---|---|---|---|---|---|---|---|
| Wildtyp SNN | 19,0 | ±2,9 | 0,31 | ±0,03 | <0,20 | 19,3 | ±2,8 |
| Linie 8 | 27,6 | | 1,25 | | 1,03 | 30,0 | |
| Linie 15 | 35,7 | | 0,73 | | 1,00 | 37,4 | |
| Linie 54 | 32,3 | | 4,60 | | 1,60 | 38,7 | |
| Linie 86 | 15,7 | | 4,47 | | 0,98 | 21,4 | |
| Linie 113 | 32,3 | | 0,71 | | 0,62 | 33,6 | |

**Tabelle B: seneszierende Blätter**

| | α-Tocopherol | | γ-Tocopherol | | α-Tocotrienol | Gesamt Vitamin E | |
|---|---|---|---|---|---|---|---|
| Wildtyp SNN | 32,9 | ±2,1 | 0,31 | ±0,05 | <0,20 | 33,1 | ±2,1 |
| Linie 8 | 50,7 | | 0,69 | | 2,69 | 54,2 | |
| Linie 15 | 54,7 | | 0,69 | | 0,81 | 56,2 | |
| Linie 54 | 37,0 | | 2,60 | | 0,35 | 40,0 | |
| Linie 86 | 36,5 | | 1,51 | | 0,43 | 38,4 | |
| Linie 113 3 | 46,2 | | 0,45 | | 2,29 | 48,9 | |

### Beispiel 8 - Klonierung eines Subfragmentes des Gens kodierend für die plastidär exprimierte Chorismat Mutase-1 aus Arabidopsis thaliana

Die DNA Sequenz kodierend für das Transitpeptid des Chorismat-Mutase-1-Gen wurde mittels polymerase chain reaction (PCR) aus *Arabidopsis thaliana* unter Verwendung eines sense spezifischen Primers (CM-1TP 5' SEQ ID Nr. 11) und eines antisense spezifischen Primers (CM-1TP 3' SEQ ID NO. 12) amplifiziert.

Die PCR Bedingungen waren die folgenden:
Die PCR erfolgte in einem 50 µl Reaktionsansatz in dem enthalten war:
- 2 µl einer *Arabidopsis thaliana* cDNA
- 0,2 mM dATP, dTTP, dGTP, dCTP
- 1,5 mM Mg(OAc)₂
- 5 µg Rinderserum-Albumin
- 40 pmol CM-1TP 5'Primer
- 40 pmol CM-1TP 3'Primer
- 15 µl 3,3 x rTth DNA Polymerase XLPuffer (PE Applied Biosystems)
- 5 U rTth DNA Polymerase XL (PE Applied Biosystems)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 55°C (Annealing)
Schritt 4: 2 Minuten 72°C (Elongation)
30 Wiederholungen der Schritte 2 bis 4
Schritt 5: 10 Minuten 72°C (Post-Elongation)
Schritt 6: 4°C (Warteschleife)

Das Amplikon wurde unter Verwendung von Standardmethoden in den PCR Klonierungsvektor pCR-Script (Stratagene) kloniert. Die Identität des erzeugten Amplikons wurde durch Sequenzierung unter Verwendung eines Vektor-spezifischen Primers bestätigt.

### Beispiel 9 - Klonierung des Gens kodierend für die cytosolisch exprimierte Chorismat Mutase-2 aus Arabidopsis thaliana

Die DNA kodierend für das *Chorismat-Mutase-2-Gen* wurde mittels polymerase chain reaction (PCR) aus *Arabidopsis thaliana* unter Verwendung eines sense spezifischen Primers (CM-2 5' SEQ ID NO. 13) und eines antisense spezifischen Primers (CM-2 3' SEQ ID NO. 14) amplifiziert.

Die PCR Bedingungen waren die folgenden:
Die PCR erfolgte in einem 50 µl Reaktionsansatz in dem enthalten war:
- 2 µl einer *Arabidopsis thaliana* cDNA
- 0,2 mM dATP, dTTP, dGTP, dCTP
- 1,5 mM Mg(OAc)₂
- 5 µg Rinderserum-Albumin
- 40 pmol CM-2 5'Primer
- 40 pmol CM-2 3'Primer
- 15 µl 3, 3 x rTth DNA Polymerase XLPuffer (PE Applied Biosystems)
- 5 U rTth DNA Polymerase XL (PE Applied Biosystems)

Die PCR wurde unter folgenden Zyklus Bedingungen durchgeführt:
Schritt 1: 5 Minuten 94°C (Denaturierung)
Schritt 2: 3 Sekunden 94°C
Schritt 3: 1 Minute 55°C (Annealing)
Schritt 4: 2 Minuten 72°C (Elongation)
30 Wiederholungen der Schritte 2 bis 4
Schritt 5: 10 Minuten 72°C (Post-Elongation)
Schritt 6: 4°C (Warteschleife)

Das Amplikon wurde unter Verwendung von Standardmethoden in den PCR Klonierungsvektor pGEM-T (Promega) kloniert. Die Identität des erzeugten Amplikons wurde durch Sequenzierung unter Verwendung des M13F (-40) Primers bestätigt.

### Beispiel 10 - Erzeugung des chimären Genkonstruktes CM-1-TP-CM-2 bestehend aus der DNA Sequenz kodierend für das Transitpeptid (TP) der Chorismatmutase-1 (CM-1) und der DNA Sequenz kodierend für die Chorismatmutase-2 (CM-2)

Zur Erzeugung des chimären Gens CM-1-TP-CM-2, wurde das Plasmid pCR-Script/CM-1-TP mit den Restriktionsenzyme NcoI/SalI verdaut.

In dieses Plasmid wurde das aus dem Plasmid pGEM-Teasy/CM-2 mittels der Restriktionsenzyme NcoI/SalI isolierte DNA-Fragment der CM-2 ligiert. Die Translation dieses chimären DNA-Konstruktes (SEQ ID No. 5) (pCR-Script/AtCM-1TP-AtCM-2, Figur 4 hat die Bildung eines Fusionsproteins zur Folge, indem das Transitpeptid der CM-1 mit der CM-2 kombiniert ist (SEQ ID NO. 6).

### Beispiel 11 - Herstellung pflanzlicher Expressionskassetten enthaltend das chimäre Gen CM-1-TP-CM-2

Transgene Pflanzen wurden erzeugt, die das chimäre Gen CM-1-TP-CM2 aus A.thaliana zum einen unter Kontrolle des konstitutiven 35S-Promotor des CaMV (Blumenkohlmosaikvirus) (Franck et al., Cell 21: 285-294, 1980) und zum anderen unter Kontrolle des samenspezifischen Promotors des Legumin Gens aus Vicia faba (Kafatos et al., Nuc. Acid. Res.,14(6): 2707-2720, 1986) exprimieren.

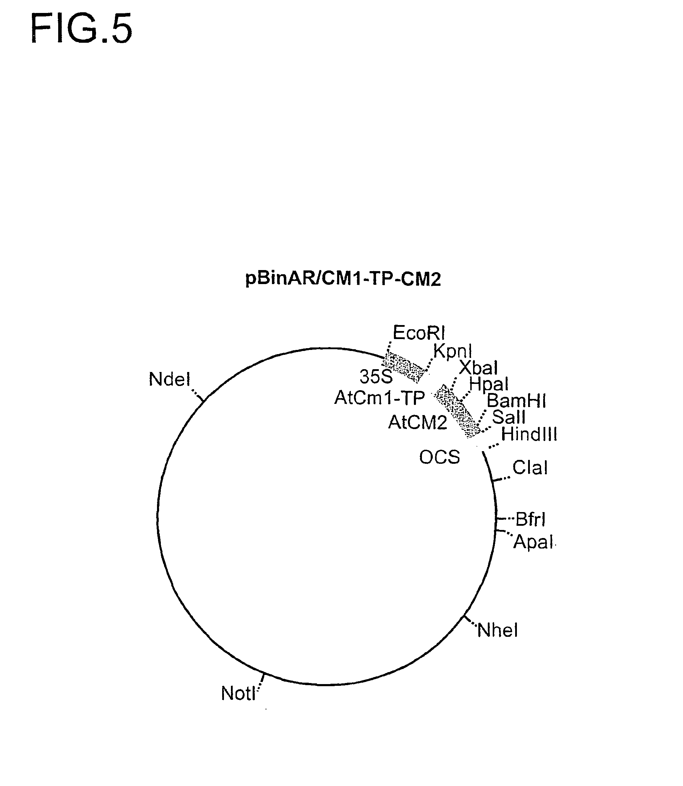

Die Grundlage des zur konstitutiven Expression des chimären Gens CM-1TP-CM-2 war der Vektor pBinAR (Höfgen und Willmitzer, Plant Sci. 66: 221-230, 1990). Dieser Vektor enthält den 35S-Promotor des CaMV (Blumenkohlmosaikvirus) (Franck et al., 1980) und das Terminationssignal des Octopin-Synthase Gens (Gielen et al., EMBO J. 3: 835-846, 1984). Zur Erstellung dieses Plasmides wurde das chimäre Gen CM-1-TP-CM2 unter Verwendung der flankierenden Restriktionsschnittstellen KpnI/SalI aus dem Plasmid pCR-Script/AtCM-ITP-AtCM-2 (Abb. 4) isoliert. Dieses Fragment wurde unter Anwendung von Standardmethoden in einen KpnI/SalI geschnittenen pBinAR ligiert. Das resultierende Plasmid (pBinAR/CM-1TP/CM-2, Abb. 5) wurde zur Erzeugung transgener *Arabidopsis thaliana* und *Nicotiana tabacum* verwendet.

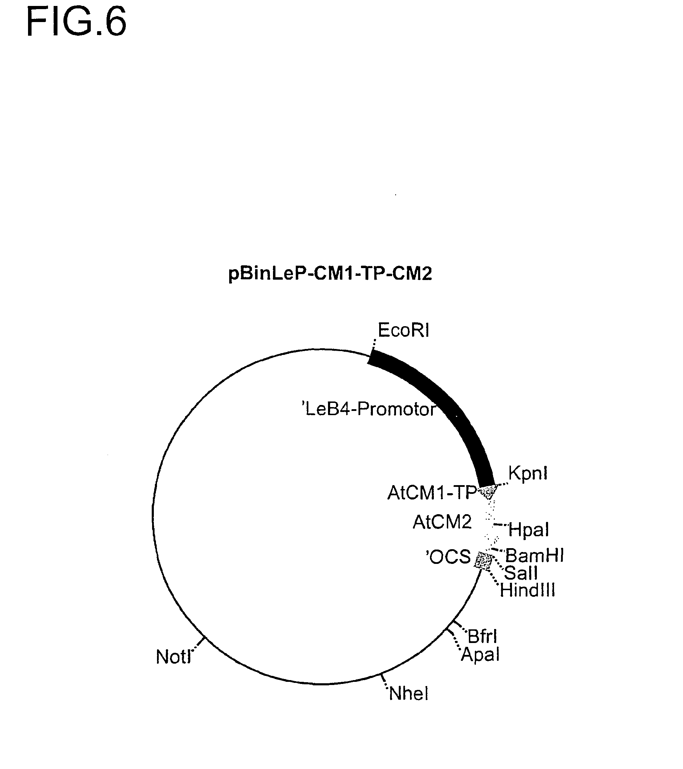

Zur Erzeugung eines Plasmides, welches die samenspezifische Expression des chimären Gens CM-1TP/CM-2 in Pflanzen ermöglicht, wurde der samenspezifiche Promotor des Legumin B4 Gens (Kafatos et al., Nuc. Acid. Res.,l4(6):2707-2720, 1986) verwendet. Aus dem Plasmid pGEMTeasy/lePNOS wurde das 2,7 Kb Fragment des Legumin B4 Gen Promotors unter Verwendung der den Promotor 5' flankierenden EcoR1 und der 3' flankierenden Kpn1 Schnittstellen isoliert. Das Plasmid pBinAR/CM-1TP/CM-2 wurde ebenfalls mit den Restriktionsenzymen EcoR1 und Kpn1 behandelt. Dies hatte zur Folge, daß der 35S-Promotor des CaMV aus diesem Plasmid herausgetrennt wurde, siehe Figur 5). Der Promotor des Legumin Gens wurde anschließend als EcoR1/Kpn1 Fragment in diesen Vektor kloniert, wodurch ein Plasmid erzeugt wurde, welches die Expression des chimären Gens CM-ITP-CM-2 unter die Kontrolle dieses samenspezifischen Promotors stellte, siehe Figur 6). Dieses Plasmid (pBinLeP/CM-1TP/CM-2) wurde zur Erzeugung transgener *Arabidopsis thaliana,* und *Nicotiana tabacum* Pflanzen verwendet.

### Beispiel 12 - Herstellung transgener Arabidopis thaliana Pflanzen, die das chimären Gen CM-1-TP-CM-2 exprimieren

Die Herstellung der Pflanzen erfolgte analog zu Beispiel 4 unter Verwendung der Plasmide (pBinAR/AtCM-1TP/CM-2) und (pBinLeP/CM-1TP/CM-2).

### Beispiel 13 - Herstellung transgener Brassica napus Pflanzen, die das tyrA-Gen exprimieren

Die Herstellung der Pflanzen erfolgte analog zu Beispiel 5 unter Verwendung der Plasmide (pBinAR/AtCM-1TP/CM-2) und (pBinLeP/CM-1TP/CM-2).

### Beispiel 14 - Herstellung transgener Nicotiana tabacum Pflanzen, die das tyrA-Gen exprimieren

Die Herstellung der Pflanzen erfolgte analog zu Beispiel 6 unter Verwendung der Plasmide (pBinAR/AtCM-1TP/CM-2) und (pBinAR1eP/CM-1TP/CM-2).

### Beispiel 15 - Charakterisierung der transgenen Pflanzen aus Beispiel 12, 13 und 14

Es wurden die Tocopherol- und Tocotrienol-Gehalte in Blätter und Samen der mit den beschriebenen Konstrukten transformierten Pflanzen *(Arabidopsis thaliana, Brassica napus* und *Nicotiana tabacum)* analysiert. Dazu wurden die transgenen Pflanzen im Gewächshaus kultiviert und Pflanzen die das Gen kodierend für die cytosolische Chorismatmutase exprimieren auf Northern- und Western Ebene analysiert. In Blättern und Samen dieser Pflanzen wurde der Tocopherolgehalt und der Tocotrienolgehalt mittels HPLC ermittelt. In allen Fällen war der Tocopherol- und/oder Tocotrienol-Gehalt in transgenen Pflanzen, die zusätzlich Chorismatmutase-Gene exprimieren, im Vergleich zu nicht transformierten Pflanzen erhöht.

## Patentansprüche

1. Verfahren zur Herstellung von Vitamin E durch Kultivierung von photosynthetisch aktiven Organismen mit einem gegenüber dem Wildtyp genetisch veränderten Shikimatweg, **dadurch gekennzeichnet, dass** man zur genetischen Veränderung des Shikimatweges mindestens eine Maßnahme ausgewählt aus der Gruppe der Maßnahmen A und B durchführt, wobei A und B folgende Bedeutung haben:
A: Erhöhung der Aktivität der Chorismatmutase des Wildtyps; B: Einbringen mindestens eines Gens in den Organismus, das für ein Protein mit einer Prephenatdehydrogenaseaktivität oder mit einer Chorismatmutaseprephenatdehydrogenaseaktivität kodiert.

2. Verfahren nach Anspruch 1, **dadurch gekennzeichnet, daß** man bei Maßnahme A die Aktivität mindestens eines Enzyms des Shikimatweges durch Überexpression von Nukleinsäuren erhöht, die Proteine mit dieser enzymatischen Aktivität codieren.

3. Verfahren nach Anspruch 2, **dadurch gekennzeichnet, daß** man eine Nukleinsäure codierend eine Chorismatmutase in den Organismus einbringt.

4. Verfahren nach Anspruch 3, **dadurch gekennzeichnet, daß** man eine Nukleinsäure codierend eine Chorismatmutase in den Organismus einbringt, deren Aktivität einer reduzierten posttranslationalen Regulation im Organismus unterliegt.

5. Verfahren nach Anspruch 4, **dadurch gekennzeichnet, daß** man eine Nukleinsäure codierend eine Chorismatmutase in den Organismus einbringt, die an der Lokalisation der Expression im Organismus einer reduzierten posttranslationalen Regulation unterliegt.

6. Verfahren nach einem der Ansprüche 1 bis 5, **dadurch gekennzeichnet, daß** man als Organismus eine Pflanze verwendet.

7. Verfahren nach Anspruch 6, **dadurch gekennzeichnet, daß** man eine cytosolische Chorismatmutase in Plastiden einer Pflanze einbringt.

8. Verfahren nach Anspruch 7, **dadurch gekennzeichnet, daß** man ein Nukleinsäurekonstrukt in die Pflanze einbringt, enthaltend eine Nukleinsäure kodierend ein plastidäres Transitpeptid und eine Nukleinsäure die ein Protein kodiert, enthaltend die Aminosäuresequenz SEQ ID NO. 4 oder eine von dieser Sequenz durch Substitution, Insertion oder Deletion von Aminosäuren abgeleitete Sequenz, die eine Homologie von mindestens 30 % auf Aminosäureebene mit der Sequenz SEQ ID NO. 4 und die enzymatische Eigenschaft einer Chorismatmutase aufweist.

9. Verfahren nach Anspruch 8, **dadurch gekennzeichnet, daß** man als Nukleinsäure, kodierend ein plastidäres Transitpeptid eine Nukleinsäure verwendet, die das plastidäre Transitpeptid einer plastidären Chorismatmutase kodiert.

10. Verfahren nach Anspruch 9, **dadurch gekennzeichnet, daß** man ein Nukleinsäurekonstrukt der Nukleinsäuresequenz SEQ ID NO. 5 in Pflanzen einbringt.

11. Verwendung eines Nukleinsäurekonstruktes, enthaltend eine Nukleinsäure kodierend ein plastidäres Transitpeptid und eine Nukleinsäure, die ein Protein kodiert, enthaltend die Aminosäuresequenz SEQ ID NO. 4 oder eine von dieser Sequenz durch Substitution, Insertion oder Deletion von Aminosäuren abgeleitete Sequenz, die eine Homologie von mindestens 30 % auf Aminosäureebene mit der Sequenz SEQ ID NO. 4 und die enzymatische Eigenschaft einer Chorismatmutase aufweist, zur Erhöhung des Gehalts an Vitamin E in photosynthetisch aktiven Organismen.

12. Verwendung eines Nukleinsäurekonstruktes, enthaltend eine Nukleinsäure kodierend ein plastidäres Transitpeptid und eine Nukleinsäure, die ein Protein kodiert, enthaltend die Aminosäuresequenz SEQ ID NO. 4 oder eine von dieser Sequenz durch Substitution, Insertion oder Deletion von Aminosäuren abgeleitete Sequenz, die eine Homologie von mindestens 30 % auf Aminosäureebene mit der Sequenz SEQ ID NO. 4 und die enzymatische Eigenschaft einer Chorismatmutase aufweist, zur Herstellung von Vitamin E in photosynthetisch aktiven Organismen.

13. Verwendung nach Anspruch 11 oder 12, **dadurch gekennzeichnet, daß** man als Nukleinsäure, kodierend ein plastidäres Transitpeptid eine Nukleinsäure verwendet, die das plastidäre Transitpeptid einer plastidären Chorismatmutase kodiert.

14. Verwendung nach Anspruch 13, **dadurch gekennzeichnet, daß** es die Nukleinsäuresequenz SEQ ID NO. 5 aufweist.

15. Verfahren nach einem der Ansprüche 1 bis 10, **dadurch gekennzeichnet, daß** man als Organismus eine Pflanze verwendet und daß man bei Maßnahme B als Gen eine Nukleinsäure codierend eine Prephenatdehydrogenase in eine Pflanze einbringt, wobei der Wildtyp zu dem Gen kein orthologes Gen aufweist.

16. Verfahren nach einem der Ansprüche 1 bis 10 und 15, **dadurch gekennzeichnet, daß** man eine Nukleinsäure codierend eine Prephenatdehydrogenase in Kombination mit einer Nukleinsäure codierend eine Chorismatmutase in eine Pflanze einbringt.

17. Verfahren nach Anspruch 16, **dadurch gekennzeichnet, daß** man eine Nukleinsäure codierend eine Chorismatmutase-Prephenatdehydrogenase in eine Pflanze einbringt.

18. Verfahren nach Anspruch 17, **dadurch gekennzeichnet, daß** man eine Nukleinsäure einbringt, die ein Protein kodiert, enthaltend die Aminosäuresequenz SEQ ID NO. 2 oder eine von dieser Sequenz durch Substitution, Insertion oder Deletion von Aminosäuren abgeleitete Sequenz, die eine Homologie von mindestens 30 % auf Aminosäureebene mit der Sequenz SEQ ID NO. 2 und die enzymatische Eigenschaft einer Chorismatmutase-Prephenatdehydrogenase aufweist.

19. Verfahren nach Anspruch 18, **dadurch gekennzeichnet, daß** man eine Nukleinsäure bakterieller Herkunft verwendet.

20. Verfahren nach Anspruch 18 oder 19, **dadurch gekennzeichnet, daß** man eine Nukleinsäure verwendet, die die in SEQ ID NO. 1 dargestellte Sequenz enthält.

21. Verwendung eines Nukleinsäurekonstruktes, enthaltend eine Nukleinsäure kodierend ein plastidäres Transitpeptid und eine Nukleinsäure, die ein Protein kodiert, enthaltend die Aminosäuresequenz SEQ ID NO. 2 oder eine von dieser Sequenz durch Substitution, Insertion oder Deletion von Aminosäuren abgeleitete Sequenz, die eine Homologie von mindestens 30 % auf Aminosäureebene mit der Sequenz SEQ ID NO. 2 und die enzymatische Eigenschaft einer Chorismatmutase-Prephenatdehydrogenase aufweist, zur Erhöhung des Gehalts an Vitamin E in photosynthetisch aktiven Organismen.

22. Verwendung eines Nukleinsäurekonstruktes, enthaltend eine Nukleinsäure kodierend ein plastidäres Transitpeptid und eine Nukleinsäure, die ein Protein kodiert, enthaltend die Aminosäuresequenz SEQ ID NO. 2 oder eine von dieser Sequenz durch Substitution, Insertion oder Deletion von Aminosäuren abgeleitete Sequenz, die eine Homologie von mindestens 30 % auf Aminosäureebene mit der Sequenz SEQ ID NO. 2 und die enzymatische Eigenschaft einer Chorismatmutase-Prephenatdehydrogenase aufweist, zur Herstellung von Vitamin E in photosynthetisch aktiven Organismen.

23. Verwendung der Nukleinsäurekonstrukte wie in einem der Ansprüche 11 bis 14, 21 und 22 definiert zur Herstellung von transgenen Pflanzen.

24. Verwendung nach Anspruch 23, **dadurch gekennzeichnet, daß** die transgene Pflanze gegenüber dem Wildtyp einen erhöhten Gehalt an Vitamin E aufweist.

25. Verwendung nach Anspruch 24, **dadurch gekennzeichnet, daß** die transgene Pflanze gegenüber dem Wildtyp eine erhöhte Resistenz gegenüber abiotischem Streß aufweist.

## Claims

1. A process for the production of vitamin E by culturing photosynthetically active organisms whose shikimate pathway is genetically modified over that of the wild type, wherein at least one measure selected from the group of measures A and B is carried out for genetically modifying the shikimate pathway, A and B having the following meanings:
A: increasing the activity of the chorismate mutase of the wild type;
B: introducing at least one gene into the organism, which gene codes for a protein with a prephenate dehydrogenase activity or with a chorismate mutase-prephenate dehydrogenase activity.

2. The process according to claim 1, wherein, in measure A, the activity of at least one enzyme of the shikimate pathway is increased by overexpressing nucleic acids which encode proteins with this enzymatic activity.

3. The process according to claim 2, wherein a nucleic acid encoding a chorismate mutase is introduced into the organism.

4. The process according to claim 3, wherein a nucleic acid encoding a chorismate mutase whose activity is subject to reduced post-translational regulation in the organism is introduced into the organism.

5. The process according to claim 4, wherein a nucleic acid encoding a chorismate mutase which is subject to reduced post-translational regulation in the organism at the localization of expression is introduced into the organism.

6. The process according to any of claims 1 to 5, wherein the organism used is a plant.

7. The process according to claim 6, wherein a cytosolic chorismate mutase is introduced into plastids of a plant.

8. The process according to claim 7, wherein a nucleic acid construct is introduced into the plant, comprising a nucleic acid encoding a plastidial transit peptide and a nucleic acid which encodes a protein comprising the amino acid sequence SEQ ID No.4 or a sequence derived from this sequence by substitution, insertion or deletion of amino acids, which has at least 30% homology at the amino acid level with the sequence SEQ ID No.4 and which has the enzymatic property of a chorismate mutase.

9. The process according to claim 8, wherein a nucleic acid which encodes the plastidial transit peptide of a plastid chorismate mutase is used as the nucleic acid encoding a plastidial transit peptide.

10. The process according to claim 9, wherein a nucleic acid construct of the nucleic acid sequence SEQ ID No.5 is introduced into plants.

11. The use of a nucleic acid construct comprising a nucleic acid encoding a plastidial transit peptide and a nucleic acid which encodes a protein comprising the amino acid sequence SEQ ID No.4 or a sequence derived from this sequence by substitution, insertion or deletion of amino acids, which has at least 30% homology at the amino acid level with the sequence SEQ ID No.4 and which has the enzymatic property of a chorismate mutase for increasing the vitamin E content in photosynthetically active organisms.

12. The use of a nucleic acid construct comprising a nucleic acid encoding a plastidial transit peptide and a nucleic acid which encodes a protein comprising the amino acid sequence SEQ ID No. 4 or a sequence derived from this sequence by substitution, insertion or deletion of amino acids, which has at least 30% homology at the amino acid level with the sequence SEQ ID No. 4 and which has the enzymatic property of a chorismate mutase, for the preparation of vitamin E in photosynthetically active organisms.

13. The use according to claim 11 or 12, wherein a nucleic acid which encodes the plastidial transit peptide of a plastid chorismate mutase is used as the nucleic acid encoding a plastidial transit peptide.

14. The use according to claim 13, which comprises the nucleic acid sequence SEQ ID No.5.

15. The process according to any of claims 1 to 10, wherein the organism used is a plant and wherein, when carrying out measure B, a nucleic acid encoding a prephenate dehydrogenase is introduced into a plant as the gene, there existing no orthologous gene to this gene in the wild type.

16. The process according to any of claims 1 to 10 and 15, wherein a nucleic acid encoding a prephenate dehydrogenase in combination with a nucleic acid encoding a chorismate mutase is introduced into a plant.

17. The process according to claim 16, wherin a nucleic acid encoding a chorismate mutase-prephenate dehydrogenase is introduced into a plant.

18. The process according to claim 17, wherein a nucleic acid is introduced which encodes a protein comprising the amino acid sequence SEQ ID No.2 or a sequence derived from this sequence by substitution, insertion or deletion of amino acids, which has at least 30% homology at the amino acid level with the sequence SEQ ID No.2 and which has the enzymatic property of a chorismate mutase-prephenate dehydrogenase.

19. The process according to claim 18, wherein a nucleic acid of bacterial origin is used.

20. The process according to claim 18 or 19, wherein a nucleic acid is used which comprises the sequence shown in SEQ ID No.1.

21. The use of a nucleic acid construct, comprising a nucleic acid encoding a plastidial transit peptide and a nucleic acid which encodes a protein comprising the amino acid sequence SEQ ID No.2 or a sequence derived from this sequence by substitution, insertion or deletion of amino acids, which has at least 30% homology at the amino acid level with the sequence SEQ ID No.2 and which has the enzymatic property of a chorismate mutase-prephenate dehydrogenase for increasing the vitamin E content in photosynthetically active organisms.

22. The use of a nucleic acid construct, comprising a nucleic acid encoding a plastidial transit peptide and a nucleic acid which encodes a protein comprising the amino acid sequence SEQ ID No.2 or a sequence derived from this sequence by substitution, insertion or deletion of amino acids, which has at least 30% homology at the amino acid level with the sequence SEQ ID No.2 and which has the enzymatic property of a chorismate mutase-prephenate dehydrogenase, for the preparation of vitamin E in photosynthetically active organisms.

23. The use of the nucleic acid constructs as defined in any of claims 11 to 14, 21 and 22 for generating transgenic plants.

24. The use according to claim 23, wherein the vitamin E content of the transgenic plant is increased over that of the wild type.

25. The use according to claim 24, wherein the resistance to abiotic stress of the transgenic plant is increased over that of the wild type.

## Revendications

1. Procédé de production de vitamine E par culture d'organismes à activité de photosynthèse avec une voie de shikimate génétiquement modifiée vis-à-vis du type sauvage, **caractérisé en ce que**, pour la modification génétique de la voie du shikimate, on entreprend au moins une mesure choisie dans le groupe des mesures A et B, où A et B ont les significations suivantes :
A : augmentation de l'activité de la chorismate mutase du type sauvage,
B : introduction dans l'organisme d'au moins un gène codant pour une protéine ayant une activité de préphénate déshydrogénase ou une activité de chorismate mutase - préphénate déshydrogénase.

2. Procédé suivant la revendication 1, **caractérisé en ce que**, dans la mesure A, on augmente l'activité d'au moins une enzyme de la voie du shikimate par surexpression d'acides nucléiques codant pour la protéine ayant cette activité enzymatique.

3. Procédé suivant la revendication 2, **caractérisé en ce que** l'on introduit dans l'organisme un acide nucléique codant pour une chorismate mutase.

4. Procédé suivant la revendication 3, **caractérisé en ce que** l'on introduit dans l'organisme un acide nucléique codant pour une chorismate mutase, dont l'activité est soumise dans l'organisme à une régulation post-translationnelle réduite.

5. Procédé suivant la revendication 4, **caractérisé en ce que** l'on introduit dans l'organisme un acide nucléique codant pour une chorismate mutase, qui est soumise, à la localisation de l'expression dans l'organisme, à une régulation post-translationnelle réduite.

6. Procédé suivant l'une quelconque des revendications 1 à 5, **caractérisé en ce que** l'on utilise un végétal en tant qu'organisme.

7. Procédé suivant la revendication 6, **caractérisé en ce que** l'on introduit une chorismate mutase cytosolique dans les plastides d'un végétal.

8. Procédé suivant la revendication 7, **caractérisé en ce que** l'on introduit dans le végétal une construction d'acide nucléique contenant un acide nucléique codant pour un peptide de transit plastidaire et un acide nucléique codant pour une protéine contenant la séquence d'acides aminés SEQ ID n° 4 ou une séquence dérivée de cette séquence par substitution, insertion ou délétion d'acides aminés, présentant une homologie d'au moins 30 % au niveau des acides aminés avec la séquence SEQ ID n° 4 et présentant la propriété enzymatique d'une chorismate mutase.

9. Procédé suivant la revendication 8, **caractérisé en ce que** l'on utilise en tant qu'acide nucléique codant pour un peptide de transit plastidaire un acide nucléique codant pour le peptide de transit plastidaire d'une chorismate mutase plastidaire.

10. Procédé suivant la revendication 9, **caractérisé en ce que** l'on introduit dans des végétaux une construction d'acide nucléique de la séquence d'acides nucléiques SEQ ID n° 5.

11. Utilisation d'une construction d'acide nucléique contenant un acide nucléique codant pour un peptide de transit plastidaire et un acide nucléique codant pour une protéine, contenant la séquence d'acides aminés SEQ ID n° 4 ou une séquence dérivée de cette séquence par substitution, insertion ou délétion d'acides aminés, présentant une homologie d'au moins 30 % au niveau des acides aminés avec la séquence SEQ ID n° 4 et présentant la propriété enzymatique d'une chorismate mutase, pour augmenter la teneur en vitamine E dans des organismes à activité de photosynthèse.

12. Utilisation d'une construction d'acide nucléique contenant un acide nucléique codant pour un peptide de transit plastidaire et un acide nucléique codant pour une protéine, contenant la séquence d'acides aminés SEQ ID n° 4 ou une séquence dérivée de cette séquence par substitution, insertion ou délétion d'acides aminés, présentant une homologie d'au moins 30 % au niveau des acides aminés avec la séquence SEQ ID n° 4 et présentant la propriété enzymatique d'une chorismate mutase, pour la production de vitamine E dans des organismes à activité de photosynthèse.

13. Utilisation suivant la revendication 11 ou 12, **caractérisée en ce que** l'on utilise en tant qu'acide nucléique codant pour un peptide de transit plastidaire un acide nucléique codant pour le peptide de transit plastidaire d'une chorismate mutase plastidaire.

14. Utilisation suivant la revendication 13, **caractérisée en ce qu'**elle présente la séquence d'acides nucléiques SEQ ID n° 5.

15. Procédé suivant l'une quelconque des revendications 1 à 10, **caractérisé en ce que** l'on utilise un végétal en tant qu'organisme et que, dans la mesure B, on introduit comme gène dans un végétal un acide nucléique codant pour une préphénate déshydrogénase, le type sauvage ne présentant pas de gène orthologue au gène.

16. Procédé suivant l'une quelconque des revendications 1 à 10 et 15, **caractérisé en ce que** l'on introduit dans un végétal un acide nucléique codant pour une préphénate déshydrogénase en combinaison avec un acide nucléique codant pour une chorismate mutase.

17. Procédé suivant la revendication 16, **caractérisé en ce que** l'on introduit dans un végétal un acide nucléique codant pour une chorismate mutase - préphénate déshydrogénase.

18. Procédé suivant la revendication 17, **caractérisé en ce que** l'on introduit un acide nucléique codant pour une protéine contenant la séquence d'acides aminés SEQ ID n° 2 ou une séquence dérivée de cette séquence par substitution, insertion ou délétion d'acides aminés, présentant une homologie d'au moins 30 % au niveau des acides aminés avec la séquence SEQ ID n° 2 et présentant la propriété enzymatique d'une chorismate mutase - préphénate déshydrogénase.

19. Procédé suivant la revendication 18, **caractérisé en ce que** l'on utilise un acide nucléique d'origine bactérienne.

20. Procédé suivant la revendication 18 ou 19, **caractérisé en ce que** l'on utilise un acide nucléique contenant la séquence représentée dans SEQ ID n° 1.

21. Utilisation d'une construction d'acide nucléique contenant un acide nucléique codant pour un peptide de transit plastidaire et un acide nucléique codant pour une protéine, contenant la séquence d'acides aminés SEQ ID n° 2 ou une séquence dérivée de cette séquence par substitution, insertion ou délétion d'acides aminés, présentant une homologie d'au moins 30 % au niveau des acides aminés avec la séquence SEQ ID n° 2 et présentant la propriété enzymatique d'une chorismate mutase - préphénate déshydrogénase, pour augmenter la teneur en vitamine E dans des organismes à activité de photosynthèse.

22. Utilisation d'une construction d'acide nucléique contenant un acide nucléique codant pour un peptide de transit plastidaire et un acide nucléique codant pour une protéine, contenant la séquence d'acides aminés SEQ ID n° 2 ou une séquence dérivée de cette séquence par substitution, insertion ou délétion d'acides aminés, présentant une homologie d'au moins 30 % au niveau des acides aminés avec la séquence SEQ ID n° 2 et présentant la propriété enzymatique d'une chorismate mutase - préphénate déshydrogénase, pour la production de vitamine E dans des organismes à activité de photosynthèse.

23. Utilisation de la construction d'acide nucléique comme défini dans l'une quelconque des revendications 11 à 14, 21 et 22, pour la production de végétaux transgéniques.

24. Utilisation suivant la revendication 23, **caractérisée en ce que** le végétal transgénique présente une teneur en vitamine E supérieure à celle du type sauvage.

25. Utilisation suivant la revendication 24, **caractérisée en ce que** le végétal transgénique présente vis-à-vis du type sauvage une meilleure résistance au stress abiotique.
